Europäisches Patentamt

European Patent Office

Office européen des brevets

(11) Publication number: **0 241 140 B1**

## EUROPEAN PATENT SPECIFICATION

(45) Date of publication of patent specification: **19.02.92**

(51) Int. Cl.⁵: **G01N 33/543**, G01N 33/566, G01N 33/569

(21) Application number: **87302025.9**

(22) Date of filing: **10.03.87**

(54) **Assay method with a multivalently labelled reagent, and means therefor.**

(30) Priority: **11.03.86 DK 1129/86**

(43) Date of publication of application:
**14.10.87 Bulletin 87/42**

(45) Publication of the grant of the patent:
**19.02.92 Bulletin 92/08**

(84) Designated Contracting States:
**AT BE CH DE ES FR GB IT LI LU NL SE**

(56) References cited:
| | |
|---|---|
| EP-A- 0 074 520 | EP-A- 0 077 671 |
| EP-A- 0 155 224 | EP-A- 0 170 746 |
| GB-A- 2 041 517 | US-A- 3 853 987 |

PATENT ABSTRACTS OF JAPAN, vol. 6, no. 34; (P-104)

PATENT ABSTRACTS OF JAPAN, vol. 9, no. 161; (P-370)

(73) Proprietor: **BioCarb AB**
**Sölvegatan 41**
**S-223 70 Lund(SE)**

(72) Inventor: **Nilsson, Hans Bertil Sture**
**Storkvagen 10**
**S-23700 Bjarred(SE)**
Inventor: **Nilsson, Kurt Gosta Ingemar**
**Andjaktsvagen 6**
**S-22253 Lund(SE)**
Inventor: **Larsson, Birgit Ann-Charlott**
**Lojtnantsvagen 21**
**S-23700 Bjarred(SE)**
Inventor: **Pahlsson, Karin**
**Savstaholmsgatan 18**
**S-21224 Malmo(SE)**
Inventor: **Ohgren, Yvonne Elisabeth**
**Svenskavagen 51**
**S-22239 Lund(SE)**

(74) Representative: **Burman, Tore et al**
**Awapatent AB Box 45086**
**S-104 30 Stockholm(SE)**

Rank Xerox (UK) Business Services

## Description

This invention relates to a method of detecting sample components; more particularly, it relates to a method of detecting the presence in a sample of cell, virus, plasma protein, hormone or antibody, or an antibody thereto, as well as a macromolecular water-insoluble carrier and a diagnostic composition suitable for use in such a method.

Chemical measurements based on immune reactions, immune-like reactions (e.g. lectin-carbohydrate interactions) or complex-forming reactions have been used for many years (1, 2) as useful tools in the analysis of the presence and quantity of proteins, carbohydrates, antigens and antibodies in biological material. Until recently, the most widely employed immunoassay method was a method using radiolabelled ligands such as antibodies and measuring the radioactivity emitted from the formed complexes. However, the use of radioactive isotopes presents a safety hazard in the laboratory as well as disposal problems. Alternative methods have therefore been developed which aim at a similar sensitivity and specificity as the radioimmunoassay, but which use non-radioactive substances and hence do not suffer from the same disadvantages as the radioimmunoassay. These methods are principally based on the use of enzymes, fluorescence or luminescence which are all reliable and reasonably sensitive (down to ng scale). Of these methods, the enzyme-linked immunoassay is probably the most widely used, as it has the advantage of generating a coloured end product from a substrate, which product can either be detected by the naked eye or measured spectroscopically in a relatively simple and therefore economical way.

The enzyme-linked immunosorbent assay (ELISA) is based on antigenantibody reactions, in which an antigen is reacted with a first antibody immobilised on a support and a second antibody bound to an enzyme which gives rise to a coloured end product when reacted with a suitable substrate. The sensitivity of this assay has, however, not always been completely satisfactory, and various improvements of the enzyme-linked immunoassay have been attempted. As an example, EP-A-77 671 discloses a reagent for the detection of an immunological substance, which reagent comprises an antibody or an antigen bound to a carrier which is a water-soluble polymer and which is provided with a multiplicity of markers, such as flourescent substances, radioisotypes, dyes, enzymes or other antibodies. It has recently been reported (3-7) that when biotinylated enzymes are employed in the double antibody ("sandwich") system in conjuction with avidin, the sensitivity of the assay is enhanced, and this amplification system has also been described to preserve the enzyme activity better than systems comprising enzyme-antibody conjugates. Another technique is the antibody bridging technique (8) which has been reported to increase the sensitivity of the assay more than 50 fold compared to the standard procedure.

A further amplification system has been devised which uses a cyclic process to amplify the antigen-antibody reaction (8). In this system, alkaline phosphatase which is an enzyme that is commonly used for immunoassay purposes, catalyses the dephosphorylation of nicotinamide adenine dinucleotide phosphate (NADP), producing nicotinamide adenine dinucleotide (NAD) which acts as a coenzyme for alcohol dehydrogenase in the presence of an excess of the substrate ethanol to produce NADH; the reduced and oxidized nucleotides are catalytically cycled back and forth by diaphorase whereby the second substrate para-iodonitrotetrazolium violet forms a formazan dye in amplified amounts. Thus, the sensitivity of the system can be increased by a factor of 250, and as little as $10^{-18}$ moles of alkaline phosphatase has been detected in this system. The present inventors, however, have found that although a good sensitivity and reproducibility is obtained by this amplification method when proteins are analysed, the sensitivity is not sufficient for analysing the presence of pathogenic organisms, e.g. bacteria, viruses or fungi, or components thereof in, for instance, pathophysiological samples.

A novel method has therefore been developed in which a substance, such as an enzyme, which directly or indirectly is capable of generating a detectable end product, is combined with the reagent for the component in the sample for which the detection system is intended, in such a way that the substance is present in multivalent form in the detection system.

The present invention provides a method of detecting the presence in a sample of a cell, virus, plasma protein, hormone or antibody, or an antibody thereto, characterised in that it comprises:

(a) contacting the sample with a first reagent bound to a solid support so that the reagent binds to the cell, virus, plasma protein, hormone or antibody, or antibody thereto;

(b) contacting the resulting sample containing the cell, virus, plasma protein, hormone or antibody, or antibody thereto bound to the reagent, which in turn is bound to the solid support, with a macromolecular water-insoluble carrier having a second reagent which is a carbohydrate or carbohydrate derivative and a label substance multivalently-bound thereto so that the second reagent and thus the macromolecular water-insoluble carrier and the label substance bind to the cell, virus, plasma protein, hormone or antibody, or antibody thereto;

2

(c) removing unbound second reagent and thus unbound carrier and label substance from the sample;

(d) treating the then-resulting sample with a compound which is a precursor of a detectable reaction product or which is capable of initiating a reaction cascade, the end product of which is detectable, so that a detectable reaction product is formed if the label substance is present; and

(e) detecting any detectable reaction product so formed and thereby detecting the presence of the cell, virus, plasma protein, hormone or antibody, or antibody thereto, in the sample.

The present invention also provides a macromolecular water-insoluble carrier suitable for use in such a method characterised in that it is

(a) coupled to a reagent which is a carbohydrate or carbohydrate derivative, the reagent being capable of binding a cell, virus, plasma protein, hormone or antibody, or an antibody thereto, the carrier comprising a substance multivalently-bound to the carrier, which substance is capable of initiating a reaction the product of which is detectable; or

(b) multivalently-bound to a substance to which the reagent is coupled and which is capable of initiating a reaction the product of which is detectable.

The present invention further provides a diagnostic composition suitable for use in such a method characterised in that it comprises:

(a) a first reagent bound to a solid support, which reagent is capable of binding a cell, virus, plasma protein, hormone or antibody, or an antibody thereto, and

(b) a second reagent which is a carbohydrate or carbohydrate derivative, reagent being coupled to a macromolecular water-insoluble carrier either directly or via a spacer, to which carrier a label substance is multivalently-bound, said substance being capable of initiating a reaction the product of which is detectable, or coupled to a macromolecular water-insoluble carrier via a label substance, the substance being multivalently-bound to the macromolecular water-insoluble carrier and the substance being capable of initiating a reaction the product of which is detectable.

In the present context, the term "cell component" is not only meant to indicate a specific component, i.e. a definable substance, but also a whole cell of which the component forms a part, as well as cell membranes, nuclei, chromosomes and organelles, e.g. mitochondria, composed of many such definable substances. Similarly, the term "virus component" refers to whole virus particles comprising the component in question, the virus envelope and the nucleic acids of which the interior of the virus is composed, apart from specific, definable substances present on or in the virus particle. In connection with whole microorganisms and viruses, components of particular interest will often be surface components. The term "circulating body component" is intended to indicate a component which does not form part of a cell or virus interior or surface, i.e. an extracellular component which may be present in body fluids, examples of which are plasma proteins, hormones and antibodies. In the following, all these components will often be referred to as sample components. It should be pointed out that the sample components need not necessarily be ones which are produced in nature, but may also be produced synthetically or semi-synthetically as analogues, derivatives or counterparts of naturally occurring compounds.

The term "antibody" refers to any substance formed as a response to exposure to an antigen, for instance such antibodies which are formed in a human or animal body against a pathogen. The antibody may be a polyclonal or monoclonal antibody.

The term "sample" is intended to indicate any fluid containing a material of biological origin (or a synthetic or semi-synthetic analogue or derivative thereof), such as solutions or suspensions of single components or a mixture of single components, or a suspension of cells or viruses or substructures thereof such as those indicated above. The sample preferably comprises a body fluid, e.g. blood, urine, lymph, faeces or sperm.

The reagent used in step a) may be any reagent capable of binding a sample component, i.e. a reagent for which the component shows affinity. If it is simply desired to separate all components in a sample from the fluid containing them, the binding may be unspecific, for instance an unspecific hydrophobic binding. In order to utilise the unique possibilities of obtaining a previously unknown assay specificity and sensitivity inherent in the detection method of the present invention, it is however preferred that the reagent is specific, that is, capable of binding a specific cell, virus or circulating body component so that a high degree of selectivity is obtained in the analysis of a sample. The reagent is preferably complementary to the sample component being analyzed or the sample component suspected to be present in the sample.

The reagent used in step b) may, in principle, also be any reagent which is capable of binding the sample component which is being analysed, and the reagents used in steps a) and b) may be different, provided that they are capable of binding the same component or different components on the same cell or virus or cell or virus substructure. It is, however, preferred that the reagents used in steps a) and b) are the same, and in particular that they are also specific to a certain component so that no other component in the sample will be bound, as this ensures a greater accuracy of the detection method of the invention.

The substance which is multivalently bound to the macromolecular carrier acts as a label by means of which it is possible to detect whether a sample component has become bound or not. By providing this substance in multivalent form the difficulty of obtaining a sufficient assay sensitivity arising from the limited number of detectable product molecules produced by the necessarily small number of enzymes resulting from univalent binding of reagent and substance (*in casu* enzyme), which difficulty is inherent in the known sandwich ELISA method, has been overcome, resulting in a far greater sensitivity that can be obtained by the known immunoassay systems not employing radioactive isotopes, due to the amplification of the binding effect obtained by the multivalent binding where a large number of label molecules are provided. The detection method of the present invention has therefore made it possible to detect, for instance, critical concentrations of a pathogen in a sample of a body fluid, which concentrations are not detectable by any of the known assay methods, which will appear from the detailed description of the invention below, including the examples.

The macromolecular water-insoluble carrier for the reagent and label substance may be any suitable carrier providing multiple binding sites.

As indicated above, the method of the present invention is used to detect the presence of sample components which have become bound to reagent a) and b) in the assay. Thus, the method serves to qualitatively determine the presence of a sample component (i.e. determine whether a sample component is present in a sample or not) and quantitatively measure the amount of sample component (as will be explained below). The principal use of the method of the invention is envisaged to be in the diagnostic field, to diagnose an illness caused by a specific pathogen, as an improvement of the conventional ELISA method, and to separate and/or purify a specific component from other components present in a sample.

Specific and preferred embodiments of the elements composing the composition which is employed for carrying out the method of the invention, are set down in the following detailed description of the invention.

DETAILED DESCRIPTION OF THE INVENTION

The choice of reagent to be employed in steps a) and b) of the method of the invention depends on the identity of the component, the presence of which is to be detected so that the reagent selected in each instance will be one which is capable of binding the component suspected to be present in the sample. Suitable reagents may be selected from a carbohydrate or carbohydrate derivative, a polypeptide, a protein or another cell or virus surface component or a circulating body component, an antibody to a carbohydrate or carbohydrate derivative, a polypeptide, a protein or another cell or virus surface component or a circulating body component, a metal conjugate or a single-stranded oligonucleotide. When the reagent is a carbohydrate or carbohydrate derivative, it is often a receptor for a cell, virus or circulating body component, such as a receptor for a bacterial membrane protein mediating bacterial adhesion to surfaces such as epithelial tissue which contains such receptors. In chemical terms, the carbohydrate or carbohydrate derivative may be a polysaccharide, glycolipid, neo-glycolipid, glycoprotein or neo-glycoprotein. The term "neo-" indicates that the compound is prepared synthetically to correspond to a naturally occurring compound by coupling the carbohydrate moiety to the lipid/protein moiety. Examples of useful carbohydrate receptors are:

4

## TABLE I

### Suggested receptor structures for microorganisms and toxins

| Strain (organism)/adhesin site of infection | Suggested specificity | Ref. |
|---|---|---|
| *E. coli* type 1 fimbriae | Man- | a) |
| Human urinary tract F-fimbriae | Galα1–4Galß-O  . | b) |
| Human urinary tract X-fimbriae | NeuAcα2–3Gal, GlcNAcß1-O- | c,d) |
| K88 pig, small intestine | Galα1–3Gal-α1-O- | e) |
| K99, calf, small intestine | GalNac-, NeuAc | f) |
| *Shigella toxin* | Galα1–4Gal- terminal position | g) |
| *S. saprophyticus* Human urinary tract | Galß1–4GlcNAcß-O, GlcNAcß1–4GlcNAc-OH | h) |
| *S. pneumococcus* Human respiratory tract | GlcNAcß–3Galß-O | i) |

*M. pneumoniae*
Human respiratory tract              Lactosamine 1

                                                        6          :

                                                   lactosamine j)

                                                        3

                                         NeuAco2–3Lactosamine 1

*Influenza virus*
Human respiratory tract              NeuAc-Gal                    k)

*Vibrio cholera* and                 Galβ1–3GalNAcβ1–4Galβ1–4GLc
*piliated gonococcus*                (GM$_1$)                   3

Intestine/human urinary tract                              1

                                              NeuAc      l)

---

a)  N. Firon, I. Ofek and W. Sharon, *Infect. Immun.*, *43*, 1984, p. 1088; E.H. Beachey (ed) *Bact. Adherence, Receptors and Recognition*, Vol. 8, Chapman and Hall, N.Y., 1980.

b)  H. Leffler and C. Svanborg-Ed'en, *FEMS Microbiol. Letters 8*, 1980,p. 127; G. Källenius, R. Möllby, S.B. Svensson, J. Winberg, A. Lundblad, S. Svensson, and B. Cedergren, *FEMS Microbiol. Letters 7*, 1980, p. 297.

c)  I. Parkkinen, J. Finne, M. Achtman, V. Vaisänen, and T.K. Korhonen, *Biochem. Biophys. Res. Commun. 111*, 1983, p. 456.

d)  V. Väisänen-Rhen, T.K. Korhonen, and J. Finne, *FEBD Lett. 159*, 1983, p. 233.

e)  M.J. Anderson, J.S. Whitehead, and Y.S. Kim, *Infect. and Immun. 29*, 1980, p.897.

f)  M. Bertolini and W. Pigman, *Carbohydr. Res.*, *14*, 1978, p. 53.

g)  J.E. Brown, K.-A. Karlsson, A. Lindberg, N. Strömberg, and J. Thurin in M.A. Chester, D. Heinegård, A. Lundblad, and S. Svensson (eds), *Proc. 7th Int. Symp. Glycoconjugates*, Lund., 1983, p. 678.

h) A. Gunnarsson, P.-A., Mårdh. A. Lundblad, S. Svensson, *Infect. Immun.*, 45, 1984, p. 41.

i) C. Svanborg-Edén, B. Andersson, L. Hagberg, H. Leffler, G. Magnusson, G. Moori, J. Dahmén, and T. Söderström, *Ann. N.Y. Acad. Sci.*, 409, 1983, p. 560.

j) T. Feizi and R.A. Childs, *Trends. Biochem. Sci.*, 10, 1985, p. 24.

k) G. F. Springer and R. R. Desai, *Ann. Clin. Lab. Sci.*, 1985, p. 294

l) J. Holmgren, *Nature*, 292, 1981, p. 431.

For the sake of greater clarity, a list of suitable reagents for different sample components is shown below.

TABLE II

Combinations of sample components and reagents

| 1<br>Sample component (component suspected to be present in the sample | 2<br>Reagent$_a$) | 3<br>Reagent$_b$) |
|---|---|---|
| 1. pathogen: | | |
| a) surface antigen | Antibody | Antibody<br>Carbohydr. receptor |
| b) surface (poly)peptide protein | Carbohydr. receptor | Antibody<br>Carbohydr. receptor |
| c) toxin | Antibody, receptor | Antibody<br>Carbohydr. receptor |
| 2. non-pathogen: | | |
| a) surface antigen | Antibody | Antibody<br>Carbohydr. receptor |
| b) surface (poly)peptide protein | Carbohydr. receptor | Carbohydr. receptor<br>Antibody |

| | | |
|---|---|---|
| 3. Carbohydrate or derivative | Antibody | Antibody |
| 4. Polypeptide or protein | Antibody or carbohydr. receptor | Antibody or carbohydrate receptor |
| 5. Other component: chromosome | Compl. single-stranded DNA | Compl. single-stranded DNA Antibody Carbohydr. receptor |
| mitochondria | Antibody | Antibody |
| cell nuclei | Antibody | Antibody |
| cell membrane | Antibody or carbohydr. receptor | Antibody or carbohydrate receptor |
| 6. Antibody | Polypeptide, protein, carbohydrate or carbohydr. der. | Polypeptide, protein, carbohydrate or carbohydrate derivative |
| 7. Single-stranded oligonucleotide | Complete single-stranded oligonucleotide | |

The solid support to which the reagent used in step a) is bound should comprise a polymer. This means that the support in itself may consist of a material to which the reagent may be bound, or the material may be coated with said polymer. In the latter case the solid support comprises a matrix of a stable solid material, such as glass, paper, cardboard or plastic, coated with the polymer. The polymer to which the reagent is bound may be selected from a plastic, a polysaccharide, a silicon polymer, an ion exchange resin, a polypeptide, silica or a silicate such as borosilicate, or cellulose. Examples of a suitable plastic comprise polystyrene, polyvinylchloride, polyethylene, polyurethane, latex, nylon, teflon, dacron, polyvinylacetate, polyvinylalcohol or any suitable copolymer thereof; examples of a polysaccharide include agarose or dextran; examples of ion exchange resins comprise both cation and anion exchange resins such as sulphonated cross-linked polymer of styrene and p-divinylbenzene (cation exchange resin) or a styrene/p-divinylbenzene co-polymer with trimethylamine groups (anion exchange resin); examples of polypeptides comprise polybrene; examples of silicon polymers include siloxane; examples of silicates include glass. If the polymer cannot be utilized directly, it may be provided with functional groups which are optionally modified to adapt it for binding a particular reagent.

The physical shape of the solid support will depend on the intended use of the support in the method of the invention. Thus, the support may be in the form of a plate, film, macroparticle, dipstick, filter or paper. Specific embodiments of the solid support include glass or plastic, optionally coated, thin layer or microtiter plates and dipsticks, polymeric macroparticles suitably packed in a chromatographic column, glass or plastic filters of a sufficient pore size to permit passage of the macromolecular carrier, and filter paper. It should be noted that for certain types of paper and for certain applications, it may not be necessary to provide the support with a reagent in order to effect unspecific binding.

For most practical applications, the sample includes, or is suspected to include, a pathogen, a non-pathogen, a carbohydrate or carbohydrate derivative, a polypeptide, a protein or another cell or virus component, an antibody to any of these components,or a single-stranded oligonucleotide which is complementary to that used as the reagent. The method of the present invention is at present believed to be particularly well suited to detect the presence of pathogens in a sample as it has been found possible to detect the presence of even low concentrations of pathogens corresponding to the concentrations in which they occur in patho-physiological samples so that it has now become possible to reliably diagnose the cause of a pathological condition.

Pathogens which are detectable by the method of the invention include bacteria, viruses, fungi (including yeasts), mycoplasma, prions and bacterial toxins. Specific examples from each group of pathogens are listed below.

1. Pathogens:

| | |
|---|---|
| a. bacteria: | *Pneumococci, Enterobacteria (E. coli, Salmonella* spp., *Shigella* spp.), *Bacillus* spp., *Clostridium* spp., *Neisseria* spp.,*Streptococcus* spp., *Staphylococcus* spp.,*Actinomycetes* spp., *Mycobacterium* spp.,*Hemophilus* spp., *Bordetella* spp., *Nocardia* spp., *Trepenema* spp., *Richettsia* spp. and *Chlamydia* spp, |
| b. viruses, | e.g. Herpetoviridae, Picornaviridae, Myxo- and Paramyxoviridae, Arboviridae, Reoviridae, Retroviridae, Rhabdoviridae, Adenoviridae. |
| c. fungi, | e.g. Alternaria, Candida, Cephalosporium spp., Coccidia, Coccidioides, Cryptococcus, Epidermophyton, Fusarium, Geotrichum, Histoplasma, Microsporum, Scopulardiopsis, Sporotrichum, Torulopsis, Trichophyto, etc. |
| d. mycoplasma, | e.g. *Mycoplasma* spp. |
| e. prions | |
| f. bacterial toxins, | e.g. Shiga toxin and Cholera toxin. |

Apart from the diagnostic utility of the method of the invention, it may also be employed to separate certain components form other components in a sample for the purposes of analysis (e.g. to check whether a recombinant organism expresses an inserted gene) or purification. These components may include non-pathogenic organisms, carbohydrates or derivatives thereof as exemplified above, either alone or as present on a cell or virus surface, a polypeptide or protein, again either alone or as present on a cell or virus surface, or a single-stranded oligonucleotide complementary to that used as the reagent which, in other words, acts as a DNA probe in, for instance, hybridization experiments.

The macromolecular water-insoluble carrier to which the reagent and label substance are bound, or to which the label substance carrying the reagent is bound may be a polymer containing multiple active sites to which the reagent and/or substance may be bound. The polymer is suitably a water-insoluble, long-chain polymer such as polystyrene or poly-p-divinylbenzene. The active sites are preferably selected from the group comprising hydroxyl, amino, thio, amido, cyano, imino, imido, keto, epoxy, nitro, ester and acyl groups, or halide-containing groups. If no suitable active sites are present on the polymer, these may be provided by appropriate modification such as subtitution of the functional group. When, in the method of the invention, the activated polymer has been contacted with the sample, remaining active sites are deactivated in order to avoid cross-reactions which might impair the results of the assay for a specific sample component. The carrier should be water-insoluble to facilitate the separation of the carrier from water-soluble components.

For the purpose of the present invention it is preferred that the macromolecular carrier is in the form of microbeads, as particles of a defined geometry ensure a better binding of the sample component to the reagent than a long polymer chain which may sterically interfere with binding. Furthermore, the microbeads should be sufficiently light to permit them to be kept suspended during contact with the solid support in step b) of the method of the invention as this ensures an improved distribution of the microbeads.

For the present purposes, the microbeads typically have a size of 0.05-20 $\mu$m. Size variations of the microbeads within this general range may be envisaged, dependent *inter alia* on the type of solid support used in the method of the invention.

The microbead typically comprises a polymer; this means that the bead may either be composed entirely of a suitable polymer or that it comprises a carrier material which is coated with a suitable polymer which, in accordance with the explanation given above, should be water-insoluble. The polymer may be selected from plastics, polysaccharides, copolymers, silicon polymers, glass, liposomes, cross-linked polymers or silica or silicates (e.g. borosilicate). Specific examples of suitable polymers may be the same as those stated above for the solid support. The microbeads may optionally be provided with multiple active sites; examples of such active sites are essentially the same as those stated above. If no suitable active sites are present, the polymer may be modified to provide appropriate functional groups. Examples of specific, advantageous modifications are shown in the reaction scheme below.

Latex (polystyrene 0.21-0.81μm)

Aminolatex (0.21-0.81μm)

Dynospheres (3-5μm)

In keeping with what has been indicated above, the substance which is multivalently bound to the macromolecular carrier (the label substance) may be selected from any substances which are capable of initiating a reaction the product of which is detectable, and in particular from enzymes, antibodies and blood clotting factors.

More specifically, the enzymes may be selected from any of the following classes.

Class 1: Oxidoreductases (acting on the CH-OH groups of donors)
Class 2: Transferases (transferring phosphorus-containing groups)
Class 3: Hydrolases (acting on ester bonds)
Class 4: Lyases (e.g. aldolase and carbonic anhydrase), or
Class 5: Isomerases (intramolecular transferases).

Examples of enzymes belonging to the different classes are stated below.

10

| E C number | Enzyme | Exemplary reaction |
|---|---|---|
| 1.1.1.1 | Alcohol dehydrogenase | Alcohol + NAD - aldehyde (or ketone) + NADH |
| 1.1.1.27 | Lactate dehydrogenase | L-lactate + NAD - pyruvate + NADH |
| 2.7.1.1 | Hexokinase | ATP + D-hexose - ADH + D-hexose + 6-phosphate |
| 3.1.3.1 | Alkaline phosphatase | An orthophosphoric monoester + $H_2O$ - an alcohol + orthophosphate |
| 3.2.1.1 | $\beta$-glucosidase | 1-(p-nitrophenyl)$\beta$-D-glycoside - p-nitrophenoxide + glucose |
| 4.1.1.1 | Pyruvate decarboxylase | Pyruvate - aldehyde + $CO_2$ |
| 4.2.1 | Fumarase | Fumarate - malate |
| 5.4.2 | Phosphoglucomutase | $\alpha$-glucose-1-phosphate -glucose-6-phosphate |
| 1.1.3.4 | Glucose oxidase | $\beta$-D-glucose + $O_2$ -D-glucono-$\delta$-lactone + $H_2O_2$ |
| 1.11.1.7 | (Horseradish) peroxidase | Donor + $H_2O_2$ - oxidized donor $2H_2O$ |
| 3.1.3.2 | Acid phosphatase | o-phosphoric monoester + H2O - alcohol + O-phosphate |
| 3.2.1.23 | $\beta$-Galactosidase | Gal-$\beta$-OR + $H_2O$ - GalOH + ROH |
| 3.4 | Proteases | Peptide + H2O - peptide$_1$ + peptide$_2$ |
| 1.13.12.5-7 | Luciferases | Luciferin + O2 - oxidized luciferin + $CO_2$ + Lu |
| 3.1 | Esterases | $R_1CO$-$OR_2$ - $R_1CO$-OH + $R_2OH$ |
| 3.5.1.5 | Urease | Urea + $H_2O$ - $CO_2$ + $2NH_3$ |
| 3.2.1.17 | Lysozyme | Hydrolysis of 1,4-$\beta$-linkages between N-acetylmuramic acid and 2-acetamido-2-deoxy-D-glucoseresidues in a mucopolysaccharide or mucopeptide. |

Enzymes are substances which are not directly detectable but which need a substrate to catalyse a reaction which has a detectable end product in accordance with either of the following formulae:

1) X moles of substrate generate X moles of detectable product, or

2) X moles of substrate generate n x X moles of detectable product (amplified process).

The label substance may also be any antibody which is able to effect a sufficient aggregation of an antigen to produce a detectable product. It should be noted that, when an antibody is used as the reagent in steps a) and b), this antibody should be different from the one used as the multivalently bound label substance. In other words, the antigen used for the aggregation should be one which differs from the sample component suspected to be present in the sample.

When using a blood clotting factor as the label substance, this may be a purified factor of, for instance, bovine or porcine origin, and in order to be useful in the method of the invention, the factor should preferably be one which initiates at least one further step in the blood clotting chain reaction. Such a factor may either be in activated form or be provided together with an activator such as a protease associated with a blood clotting factor. The blood clotting factor may be selected from fibrinogen (I), prothrombin (II), thrombin (IIa), tissue thromboplastin (III), proaccelerin (V), accelerator globulin (VI), proconvertin (VII), antihemophilic globulin (VIII), plasma thromboplastin component (IX), autoprothrombin III (X), plasma thromboplastin antecedent (XI), Hagemann factor (XII) and fibrin stabilizing factor (XIII).

The compound with which the label substance is reacted in step c) and which then forms the detectable reaction product may suitably be selected from blood clotting factors (other than one used as the label substance), an antigen to the antibody label causing a detectable aggregation, or an enzyme substrate which is catalyzed by the enzyme to form a coloured, fluorescent or luminescent product, or to produce a colour change (e.g. NADH (produced cyclically as described above) catalyzing thiazolyl blue to produce a colour change from yellow to blue). The enzyme-substrate reaction may also result in a decrease or increase in the colour, fluorescence or luminescence of the detectable product dependent on the concentration of sample component to be assayed, in which the case the method of the invention may be used for semiquantitative analysis. The enzyme substrate is suitably selected from phenolphthalein orthophosphate, thymophthalein phosphate, ortho-nitrophenyl glycoside, naphtholes, anilines, coumarines, tetrazolium dyes, dansyl and derivatives thereof, NADP and isothiocyanates.

DESCRIPTION OF PREFERRED EMBODIMENTS

In a preferred embodiment of the method of the invention,

a) the solid support comprises macroparticles to which the reagent is bound, which macroparticles are packed in a column, or the solid support comprises a porous filter to which the reagent is bound, and the sample is passed through the column or filter,

b) the macromolecular carrier comprises microbeads of a size of 1-20 $\mu$m to which the same or another reagent is coupled and to which an enzyme is multivalently bound, a suspension of which microbeads is passed through the column, and

1-20 $\mu$m to which the same or another reagent is coupled and to which an enzyme is multivalently bound, a suspension of which microbeads is passed through the column, and

c) a solution or suspension of enzyme substrate is passed through the column after which a colour, fluorescence or luminescence develops,

whereby the presence in the sample of any cell, virus or circulating body component which has become bound to the reagent in steps a) and b) is detected.

By using such a column, it is possible to analyse relatively large sample volumes.

In another preferred embodiment of the method of the invention,

a) the solid support comprises a dipstick or film to which the reagent is bound, which dipstick or film is immersed in the sample,

b) the dipstick or film is contacted with the macromolecular carrier comprising microbeads of a size of 0.05-10 $\mu$m to which the same or another reagent is coupled and to which an enzyme is multivalently bound, by immersing it in a suspension of microbeads, and

c) the dipstick or film becomes opaque at high cell, virus or circulating body component concentrations, or the dipstick is immersed in a solution or suspension of enzyme substrate after which a colour, fluorescence or luminescence develops,

whereby the presence in the sample of any cell, virus or circulating body component which has become bound to the reagent in steps a) and b) is detected.

It has been found that the dipstick or film becomes opaque at, for instance, a bacterial concentration of

about $10^{10}$. However, as pathologically critical bacterial concentrations are often in the range of $10^6$-$10^7$ bacteria/ml so that it will often be necessary to immerse the solid support in an enzyme substrate solution to produce a reaction the product of which is spectrometrically detectable.

In a further preferred embodiment of the method of the invention,

a) the solid support comprises a film or plate, optionally provided with wells, to which the reagent is bound, and the sample is applied on the film or plate and removed after a period of time required to bind cell, virus or circulating body components in the sample,

b) the macromolecular carrier comprises microbeads of a size of 1-20 $\mu$m to which are the same or another reagent is coupled and to which an enzyme is multivalently bound, and a suspension of the microbeads is applied on the film or plate and removed after a period of time required to bind cell, virus or circulating body components in the sample, and

c) a solution or suspension of enzyme substrate is applied on the film or plate after which a colour, fluorescence or luminescence develops,

whereby the presence in the sample of any cell, virus or circulating body components which has become bound to the reagent in steps a) or b) is detected.

The application of the sample to the film or plate (e.g. a microtiter plate) will typically be performed by placing a drop of the sample on the film or plate by means of, e.g., a pipette.

Instead of measuring the amount of sample component bound to the reagent, it may alternatively be advantageous to determine the amount of sample component which has not become bound. Thus, the invention further relates to a method as described above in which the amount of cell, virus or circulating body component bound to the reagent in step a) is quantitized by means of measuring the proportion of microbeads of a predetermined quantity which have not become bound to the cell, virus or circulating body component in question.

Preferred embodiments of the method of the invention are summarized in the reaction schemes shown below.

**Dipstick with layers of reagent and bacteria**

Carbohydrate

Bacterium with binding lectins

Spherical micro-bead (0.2-5 μm) with multivalently bound enzyme

**Column with macro-beads, modified with reagent**

Carbohydrate

Bacterium with binding lectins

Spherical micro-bead (0.2-5 μm) with multivalently bound enzyme

A specific embodiment of the method of the invention is described in the following with reference to the diagnosis of urinary tract infections.

To a large extent, urinary tract infections are caused by certain pathogenic E.coli, in particular P-fimbriated E.coli as these are able to adhere to the epithelial cell wall of the urinary tract. The adhesion is mediated by receptor-specific interactions, i.e. a filamentous polypeptide structure (called fimbriae) interact with a carbohydrate structure (Galα1-4Galβ-O-) on the surface of the epithelial cells.

The characterization of clinical isolates has hitherto been based on bioassays, for instance attachment to epithelial cells or hemagglutination. The evaluation is complicated by the fact that adhesins which are specific for several receptors may be coexpressed, and that the receptor composition of the target cells varies between individual tissues and species (cf. 13). The sensitivity of such assays is low which means that samples must be grown before analysis.

A recently proposed assay method is based on latex bead agglutination. In such assays, receptor compounds are bound to plastic beads via spacer arms. Interaction with adhesin-carrying bacteria results in agglutination. This method also lacks sensitivity, but is highly specific.

The present invention has overcome the problem of too low a sensitivity of the conventional diagnostic methods of determining the presence of pathogens in a sample by providing a diagnostic system in which

14

bacteria (or other pathogens) are concentrated on a solid support (film, dipstick or carrier material in a column) by being bound to a reagent coupled to that support. The bacteria are then contacted with spherical particles (microbeads) carrying the reagent and a multiplicity of enzymes. Beads which have not been attached are removed by washing, after which enzyme substate is added to produce a coloured, fluorescent or luminescent product or to activate a second enzyme system which further amplifies the effect of the initial bacterial binding to the solid support.

In another aspect, the present invention relates to a macromolecular carrier to which is

a) coupled a reagent which is capable of binding a cell, virus or circulating body component or an antibody thereto, the carrier comprising a substance multivalently bound to the carrier, which substance is capable of initiating a reaction the product of which is detectable, or

b) multivalently bound a substance to which said reagent is coupled and which is capable of initiating a reaction the product of which is detectable.

The carrier, reagent and substance exhibit the features described above for the method of the invention in which the carrier may be employed.

In a further aspect, the invention relates to a diagnostic composition which comprises

a) a reagent bound to a solid support, which reagent is capable of binding a cell, virus or circulating body component or an antibody thereto, and

b) the same or another reagent coupled to a substance which is multivalently bound to a macromolecular carrier and which is capable of initiating a reaction the product of which is detectable, or coupled to a macromolecular carrier which comprises a substance multivalently bound to the carrier, the substance being capable of initiating a second reaction the product of which is detectable.

The specific features of the composition are apparent from the description of the method of the invention in which a composition of this type may be employed.

In accordance with the invention, it is further contemplated that the composition may be composed of a multiplicity of dipsticks to which the reagent is bound, each dipstick or subgroup of dipsticks being provided with a different reagent, each reagent being able to bind a different cell, virus or circulating body component or an antibody thereto. Alternatively, the composition may comprise a microtiter plate provided with a multiplicity of wells to which the reagent is bound, each well or subgroup of wells containing a different reagent, each reagent being able to bind a different cell, virus or circulating body component or an antibody thereto. This means in either case that it will be possible to diagnose a wide variety of diseases by means of the the same diagnostic composition, thereby greatly simplifying the diagnostic procedure.


BRIEF DESCRIPTION OF THE DRAWINGS

The invention is described with reference to the appended drawings in which

Fig. 1 is a graph showing the sensitivity (absorbance at 405 nm) of the assay of the invention as a function of the bacterial concentration,

Fig. 2 shows the detection of P-fimbriated *E. coli* immobilized on Trysite film incubated with 1) (Gal-Gal)-$_n$-ALP (to the left) or microbeads (to the right) and 2) p-nitrophenyl phosphate, and

Fig. 3 shows the principle of enhancing the sensitivity of the assay system by means of a second, cyclic enzymatic reaction.

The invention is further described in the following non-limiting examples.


MATERIALS AND METHODS

The oligosaccharide $Gal\alpha1$-$4Gal\beta$-0-2-(carbomethoxyethylthio)ethylglycoside was synthesized according to Dahmém et al (10,11). The glycoconjugate was coupled to KLH (Keyhole Limpet Hemocyanin) (11,12) and used as an antigen to produce monoclonal antibodies in mice. The antibodies (cell line $87/5C_{11}$) were produced by Biocarb Chemicals AB, Sweden.

Latex beads 0.81 $\mu$m were obtained from Seragen Diagnostics, Indianapolis, USA. Latex beads and amino-latex beads 0.23 $\mu$m were obtained from Serva Feinbiochemica, Heidelberg, West Germany. Dynosphere beads (3 $\mu$m, tosyl-activated) were obtained from Dyno Industrier, Norway. Trysite (polystyrene) film (0.2 mm thick) was obtained from Dow Chemicals, Miss., USA and Sepharose® macrobeads 6MB, CNBr-activated from Pharmacia Fine Chemicals, Uppsala, Sweden.

Calf intestine alkaline phosphatase (ALP) was obtained from Boehringer Mannheim, West Germany, and 4-nitrophenyldisodium orthophosphate from BDH Chemicals Ltd., Poole, Great Britain. 5-Aminosalicyclic acid and o-nitrophenyl-$\beta$-D-galactopyranoside were obtained from Sigma, St. Louis, Miss., USA. Bovine serum albumin (BSA), alcohol dehydrogenase (ADH), diaphorase and p-iodonitrotetrazolium violet (INT) were all obtained from Sigma, St. Louis, Miss., USA. N-(3-Dimethylaminopropyl)-N-ethyl-carbodiimide hydrochloride was obtained from Merck-Schuchardt, West Germany. Peroxidase-conjugated rabbit immunoglobulins to mouse immunoglobulins were from DAKO-immunoglobulins A/S, Denmark and globoside from BioCarb Chemicals AB, Sweden. Gal$\alpha$1-4Gal$\beta$-0-CETE-BSA ((Gal-Gal)$_n$-BSA) and (Gal$\beta$1-4GlcNAc)$_n$-BSA were obtained from SSA, Fine Chemicals Division, Arlöv, Sweden. $\beta$-Galactosidase (grade VI from *E. coli*) and horseradish peroxidase (type VI) were obtained from Sigma, St. Louis, Miss., USA.

The *E. coli* strains selected for illustrating the principle of the diagnostic method of the invention for the detection of microorganisms were either manipulated *E. coli* strains or strains taken from the urine of patients diagnosed for pyelonephritis (for leading references, see H. Leffler and C. Svanborg-Edén, "*E. coli* lectins/adhesins binding to glycolipids", in *Bacterial Lectins*, ed. D. Mirelman, Wiley, 1985 (13)). The most frequently used strains have been characterized and found to be 04:K12:H1 (GK1) and K12:H48 (C600). Cultivation was performed on Colonization Factor Antigen (CFA) agar plates overnight at 37°C. Colonies were harvested and suspended in PBS, pH 7.4. The presence of P-fimbriae was tested with both cell-binding assays and with newly developed kits (15,16,17). *Staphylococcus saprophyticus* strains were obtained from Malmö General Hospital, Sweden, where they were isolated from urine and characterized.

Preparation of starting materials

I. *Preparation of (Gal-Gal)$_n$-ALP*

1.5 ml of a 66.7 mM Gal$\alpha$1-4Gal$\beta$-0-2-(carbomethoxyethylthio)ethylglycoside solution in ethanol was treated with 0.2 ml 75% (w/v) hydrazine in water. The solution was stirred at room temperature overnight. The solution was then evaporated to dryness. The synthesized hydrazide (7.35 mg in 200 $\mu$l DMSO) was converted to the corresponding acyl azide according to Dahmén et al. (12) and was then added dropwise to a solution of 10 mg/ml alkaline phosphatase (ALP) in 0.05M sodium borate buffer, pH 9.0. The pH was maintained at 9.1-9.3 during the reaction. The glycosylated ALP formed was dialyzed against 50 mM Tris-HCl, pH 8.0, 0.02% NaN$_3$, and stored in a refrigerator when not in use.

II a) and b). *Preparation of NH$_2$-latex and BSA-latex*

Latex beads (0.81 $\mu$m or 0.2 $\mu$m) were nitrated with fuming red nitric acid/glacial acetic acid (1:4 v/v) and reduced with sodium dithionite. The NH$_2$-latex beads formed were covalently bound to BSA according to the method described in NASA TECH BRIEF (MSC 13906) (14). The BSA-latex beads were suspended in buffer with 0.1% of NaN$_3$ and stored in a refrigerator when not in use.

Preparation of microbeads, general procedures

III. *Preparation of (Gal-Gal)$_n$-ALP bound to BSA-latex*

Two different methods were used for covalent immobilization of glycosylated ALP to latex beads - the glutaraldehyde method and the carbodiimide coupling procedure.

III:1. *Glutardialdehyde coupling*

25 $\mu$l of the glycosylated ALP solution, obtained in section I, (10 mg/ml) were mixed with 10 $\mu$l of glutardialdehyde (2.5% w/v) in 200 $\mu$l of 0.1M potassium phosphate buffer, pH 7.5. After one hour, 50 $\mu$l of a BSA-latex suspension (10$^{13}$ particles/ml, 0.21 or 0.81 $\mu$m) were added. The reaction mixture was left overnight, filtered and washed with water. The activity of the beads was tested according to the procedure described below ("Characterization of (Gal-Gal)$_n$-ALP modified latex beads").

III:2 *Carbodiimide coupling*

25 $\mu$l of a solution of glycosylated ALP, obtained in section I, (10 mg/ml) were mixed with 2.5 mg of carbodiimide (N,N-dimethylaminopropyl-N′-ethylcarbodiimide) in 125 $\mu$l of water. The pH was adjusted to 4.7, and 40 $\mu$l of a suspension of BSA-latex (10$^{13}$ particles/ml, 0.21 or 0.81 $\mu$m) were added. The reaction was stopped after 12 hours. The modified latex beads were filtered, washed with water and analyzed (see "Characterization of (Gal-Gal)$_n$-ALP modified latex beads").

### IV. *Preparation of (Gal-Gal)$_n$-ALP bound to NH$_2$-latex beads*

60 $\mu$l of a NH$_2$-latex suspension ($10^{13}$ particles/ml, 0.21-0.81 $\mu$m) were washed with water and centrifuged. The beads were suspended in 0.1 ml of water. 2 mg of (Gal-Gal)$_n$-ALP (in 175 $\mu$l of water) and 275 $\mu$l (50 mg/ml) of carbodiimide were added. The mixture was incubated on a shaker overnight. Before washing with water, the remaining NH$_2$- or carbodiimide groups on the beads were blocked by incubating the beads with a BSA (10 mg/ml) solution. NH$_2$-latex bound (Gal-Gal)$_n$-ALP was also prepared by the method described in II. In one experiment, ALP was coupled to NH$_2$-latex, followed by coating with globoside.

### V. *Preparation of latex beads with a mixture of (Gal-Gal)$_n$-BSA and ALP*

A molar mixture (1:10) of (Gal-Gal)$_n$-BSA (0.5 mg) and ALP (5 mg) in 500 $\mu$l of water was added to 300 $\mu$l of a NH$_2$-latex bead suspension ($10^{13}$ particles/ml), which had been activated with carbodiimide. The reaction was allowed to proceed at 4°C overnight. Remaining NH$_2$- and carbodiimide groups were blocked with a Tris buffer (0.1M, pH 8.0) followed by BSA (10 mg/ml). After washing the beads, they were suspended in buffer with 0.1% NaN$_3$.

### *Preparation of latex beads modified with $\beta$-galactosidase and (Gal-Gal)$_n$-BSA*

300 $\mu$l of a NH$_2$-latex suspension ($10^{13}$ particles/ml, 0.81 $\mu$m) were washed with water and centrifuged. The pellet was suspended in 1 ml of 1 M HCl and cooled to +4°C. 100 $\mu$l of a 0.05 M sodium nitrite solution were added. The beads were centrifuged after 15 minutes, washed with water and suspended in 1 ml of 50 mM potassium phosphate, pH 7.2. A mixture of (Gal-Gal)$_n$-BSA and $\beta$-galactosidase (1/100 w/w) was allowed to react with the diazotized beads. After 10 minutes, the reaction was blocked with 0.5 ml $\beta$-naphtol (1 mg/ml) solution, and the beads were centrifuged, washed and suspended in Tris buffer, pH 8.0.

### VI. *Diazo coupling of ALP (or $\beta$-galactosidase or horseradish peroxidase) and (Gal-Gal)$_n$-BSA to NH$_2$-latex*

ALP coupling

300 $\mu$l of a suspension of NH$_2$-latex ($10^{13}$ particles/ml, 0.81 $\mu$m) were washed with water and centrifuged. The pellet was suspended in 1 ml of 1M HCl and cooled to 4°C. 100 $\mu$l of a 0.05M sodium nitrite solution were added. The beads were centrifuged after 15 minutes, washed with water and suspended in 1 ml of 50 mM potassium phosphate, pH 7.2. A mixture of (Gal-Gal)$_n$-BSA and ALP (10:1 w/w) was allowed to react with the diazotized beads. After 10 minutes, the reaction was blocked with a 0.5 ml $\beta$-naphtol (1 mg/ml) solution, and the beads were centrifuged, washed and suspended in Tris buffer, pH 8.0.

$\beta$-Galactosidase or horseradish peroxidase coupling

Beads to which these enzymes were bound were produced in an analogous manner with the exception that the ration between (Gal-Gal)$_n$-BSA and $\beta$-galactosidase was 1:100 w/w instead of 10:1 w/w.

### VII. *Coating to latex (or NH$_2$-latex)*

A third method was also used in which amino-latex (see preparation of BSA-latex) or non-modified latex beads were coated with (Gal-Gal)$_n$-ALP. An excess of modified enzyme was incubated with the beads for 12 hours. The beads were filtered and washed with buffer and stored at 4°C when not in use.

### VIII. *Preparation of (Gal-Gal)$_n$-ALP bound to Dynosphere beads*

A 10% solution of tosyl-activated Dynosphere beads (3 $\mu$m, $10^{10}$ particles/ml) was allowed to react at 4°C with (Gal-Gal)$_n$-ALP (molar excess 100%) at pH 9.6 overnight. The covalently modified beads were washed several times with buffer and stored in a buffer with 0.1% NaN$_3$. A modification of this method was also used in which Dynosphere was allowed to react with the parent enzyme ALP followed with coating with globoside.

17

## IX. *Preparation of latex beads with a mixture of ALP and Gal-Gal antibodies*

Monoclonal antibodies against Gal$\alpha$1-4Gal$\beta$- were obtained from Biocarb AB, Sweden. The antibodies have been characterized as IgG antibodies. The antibodies were concentrated by precipitation with saturated ammonium sulphate, followed by purification on a DE-52 ion exchange column. The antibodies were eluted with a 0-0.4M sodium chloride/Tris buffer gradient. Collected fractions were tested for their ability to bind (Gal-Gal)$_n$-BSA.

NH$_2$-latex (0.2 $\mu$m) beads were modified with ALP/antibodies (1:10 w/w) in essentially the same way as that described under VI. The modified beads were washed and dialyzed against water and stored in a solution of 0.05M phosphate buffer, 0.1% NaN$_3$, pH 7.2.

### Preparation of macrobeads

### *Coupling of BSA to agarose beads (Sepharose® 6MB)*

60 mg of BSA were dissolved in 3 ml of 0.1M NaHCO$_3$, pH 9, and added to 6 ml of cyanogen bromide activated Sepharose® 6MB which had been rapidly washed with the above cold buffer. Coupling was carried out overnight on an end-over-end rocking table at 4°C. The beads were washed with coupling buffer and distilled water.

### *Preparation of gal$\alpha$1-4gal-$\beta$-0-BSA-agarose beads (Sepharose® 6MB)*

The BSA-agarose (1.5 g) was washed with 0.4M NaHCO$_3$, pH 9.3. The glycoside Gal$\alpha$1-4Gal$\beta$-0-CETE was converted to the corresponding acyl azide as described above. A solution of 0.3 ml of acyl azide (containing about 8 mg of the derivative) as added to a suspension of 1.5 g of BSA-agarose and 0.5 ml of 0.4M NaHCO$_3$, pH 9.3. The reaction was allowed to proceed at 20°C for 4 hours and then at 4°C overnight. The product was washed with distilled water.

### Characterization of modified enzyme and modified beads

### *Characterization of (Gal-Gal)$_n$-ALP*

The absorbance at 280 nm of the modified enzyme was related to the parent enzyme absorbance at a defined concentration. The enzyme activity was determined on an automated PRISMA analyzer (Clinicon AB, Stockholm, Sweden). The activity was related to that found in the parent enzyme. The sugar content was related to a standard dilution of Gal$\alpha$1-4Gal$\beta$-0-2-(carbomethoxyethylthioethyl)glycoside by the phenol-sulphuric acid method (M. Dubois, K.A. Gilles, J.K. Hamilton, P.A. Rebers and F. Smith, *Anal. Chem. 28*, 1956, p. 350 (19). On duplicate runs, it was found that 39-40 mole sugar/mole enzyme was covalently linked to ALP. The enzyme activity (1.4 $\mu$kat/ml) was found to be roughly the same compared with the parent enzyme (activity 1.8 $\mu$kat/ml).

### *Characterization of (Gal-Gal)$_n$-ALP modified latex beads*

The activity of a suspension of modified latex beads (0.21, 0.81 $\mu$m, carbodiimide coupling procedure) was tested by adding p-nitrophenylphosphate to the beads. The parent enzyme was used as a standard. The activity was found to correspond to 95-100 molecules ALP/particle or approximately 3700-4000 molecules Gal-Gal/particle. The methods for characterizing the other beads described above were analogous.

### *Characterization of beads modified with (Gal-Gal)$_n$-BSA and $\beta$-galactosidase or peroxidase*

The methods for testing the activity of the beads were analogous to those described in the section entitled "Characterization of (Gal-Gal)$_n$-ALP modified latex beads". 5-Aminosalicyclic acid was used as the substrate for peroxidase and o-nitrophenylgalactoside was used as the substrate for $\beta$-galactosidase.

The activity of the beads was found to correspond to 220 molecules of $\beta$-galactosidase/particle and 605 molecules of peroxidase/particle, respectively.

The beads were tested for binding of immobilized P-fimbriated *E. coli*. The results were in accordance with those obtained for ALP-bound microbeads (cf. Example 1 and Table 2) except that the sensitivity was lower.

Test of specificity of modified beads

The different beads, characterized in Table I, were tested for specificity in an agglutination test. The strains were assigned as P-fimbriated and non-P-fimbriated, respectively, by means of specific agglutination (16). The data in the Table indicate that specific binding could not be obtained for all particles. However, in the cases where specificity was not perfect for P-fimbriated *E. coli*, there was a marked difference in sensitivity of the agglutination between P-fimbriated and non-P-fimbriated strains.

EXAMPLE 1

Detection of P-fimbriated *E. coli* bound to polystyrene films coated with Gal-Gal-BSA

Transparent 0.2 mm thick Trysite films were incubated in a solution of $(Gal\alpha1-4Gal\beta)_{31}$-O-BSA (100 $\mu$g/ml) for 9 hours. The films were washed with PBS and incubated for 0.5 hour with $10^{10}$, $10^9$, $10^8$, $10^7$, $10^6$ bacteria/ml suspensions of P-fimbriated *E. coli* (isolated from the urine of patients with pyelonephritis). After washing with 0.1M Tris-0.05M NaCl buffer, pH 7.5, and water, the film was microscopically inspected for binding of bacteria to the transparent film. A high density binding was obtained with highly concentrated bacterial suspensions. Non-P-fimbriated *E. coli* suspensions only bind to the film in low quantities, if at all. Inhibition of binding to the film was confirmed by first incubating the bacterial suspension with $Gal\alpha1-4Gal\beta$-OEt (100mg/ml and 10 mg/ml, from SSA, Fine Chemicals Division, Arlöv, Sweden) and then incubating the film with the bacterial suspension. Only a few bacteria were detected on the film by visual inspection in a microscope.

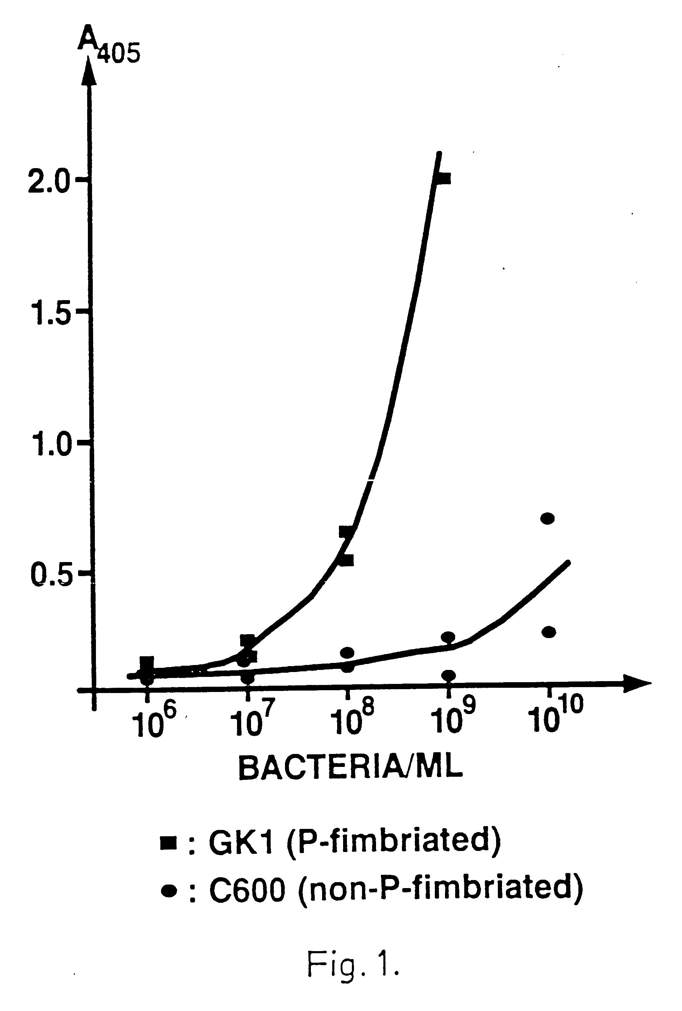

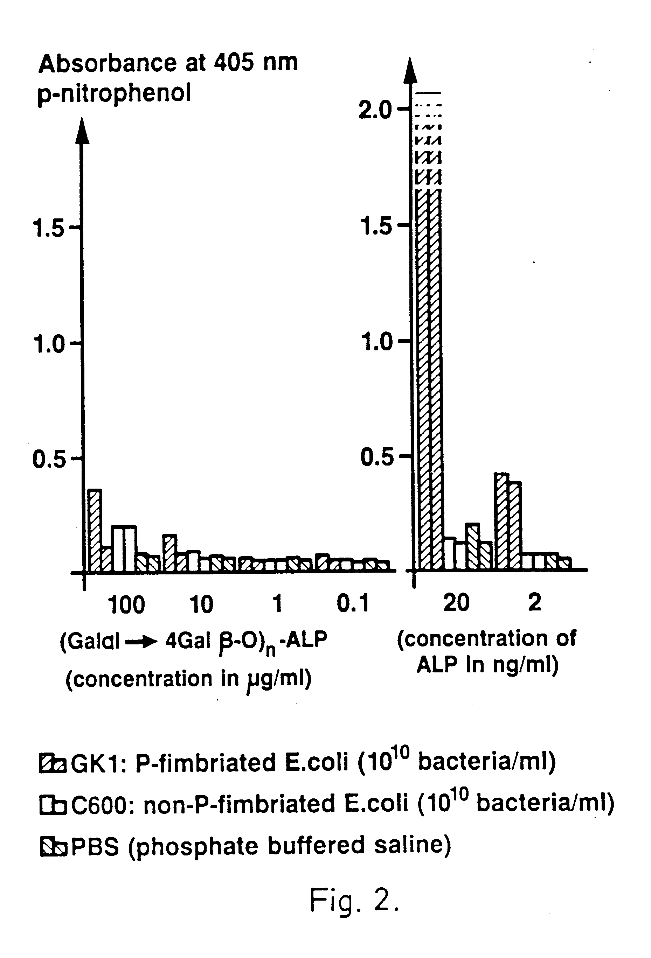

The film with bound bacteria was then incubated for 0.5 hour with a bead suspension ((Gal-Gal)$_n$-ALP modified latex, 0.81 $\mu$m), followed by washing with a Tris buffer, pH 7.5. At high bacterial concentrations ($10^{10}$ bacteria/ml), the film became opaque, which could be detected by the naked eye. At lower bacterial concentrations, the bound bacteria could be detected by reacting the bound enzyme beads with the substrate p-nitrophenylphosphate (10 mM in 1M diethanolamine buffer, pH 9.8, containing 0.5 mM $MgCl_2$). Fig. 1 shows the absorbance at 405 nm as a function of bacterial concentration (two *E. coli* strains: GK1 and C600) from triple runs. The detection limit with p-nitrophenylphosphate as a substrate was $10^6$-$10^7$ bacteria/ml. The experiment was repeated in an analogous way by use of an ELISA microtiter plate instead of Trysite film. Although the surface and the volume of the cells are limiting factors, it was possible to visualize bacterial concentrations down to $10^8$ bacteria/ml. The enhancement of the detection is apparent when comparing the above results with those obtained with the known sandwich-ELISA technique (20). If the microbead incubation step is replaced by incubation with $(Gal\alpha1-4Gal\beta)_n$-ALP solution followed (after washing) by incubation with the substrate, it was not even possible to detect any presence of immobilized bacteria at a concentration of $10^{10}$ bacteria/ml (Fig. 2). The background absorbance was also higher in this experiment compared to the experiment where microbeads were used. The detection limit for specific particle agglutination (16) is in the range of $10^8$-$10^9$ bacteria/ml.

EXAMPLE 2

Detection of P-fimbriated *E. coli* (GK1) bound to Gal-Gal BSA-agarose beads

Gal-Gal conjugated BSA-agarose and BSA-agarose (0.5 ml) (control) were packed in three columns (8 mm x 50 mm) (two containing Gal-Gal BSA and one containing BSA-agarose) consisting of PVC plastic tubes provided with a porous glass filter at the bottom. The columns were washed with PBS (phosphate buffered saline), pH 7.2. A suspension of *E. coli* strain GK1 (0.3 ml, OD = 1.03), resuspended in PBS, was applied to the BSA-column and to one of the Gal-Gal-BSA-columns. A suspension of the non-P-fimbriated *E. coli* strain C600 (0.3 ml, OD-1.40) was applied to the second Gal-Gal-BSA-column.

After 5 minutes, the gels were washed with PBS (3x0.9 ml). The eluents were collected and OD-determined. It was found that 16% of the GK1 added were retained on the Gal-Gal-BSA-column. Strain C600 was completely eluted out from the column. The GK1 strain was not found to adhere to the BSA-agarose column.

Directly after the elution step, 200 $\mu$l of a microbead solution (3 $\mu$m Dynosphere beads modified with (Gal-Gal)$_n$-ALP, $10^4$ active ALP/particle) were added to each column. After 5 minutes, the columns were washed successively with PBS (3x0.9 ml), water (3 ml) and diethanolamine containing 1mM $MgCl_2$ (3 ml).

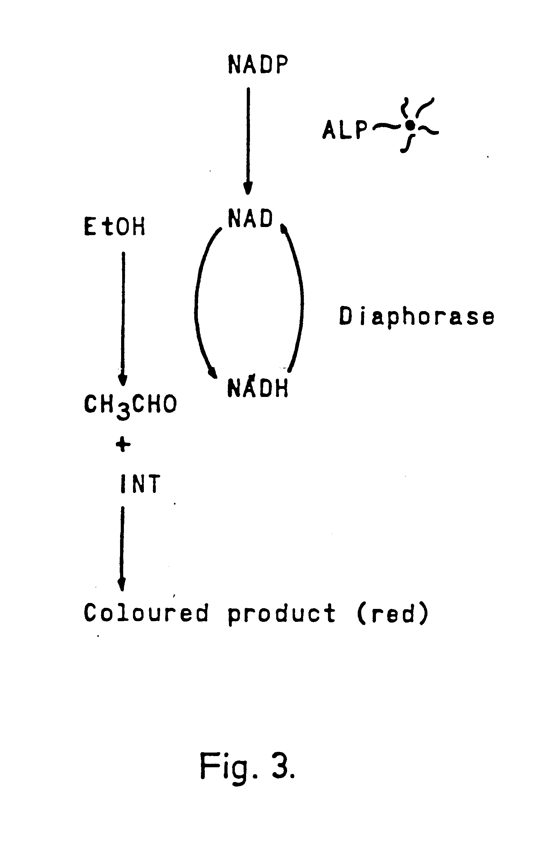

The enzyme-linked microbeads were made detectable by adding 200 $\mu$l of a 0.1mM p-nitrophenylphosphate solution. The columns with the adsorbed *E. coli* strain GK1 turned yellow, while the other two columns were unchanged, which was observed after 20 minutes of incubation. The experiment was repeated with dilutions of strain GK1. A dilution corresponding to $10^6$ bacteria/mlcould be visualized by spectrophotometric detection of formed p-nitrophenyl at 405 nm. When nicotinamide adenine dinucleotide phosphate (NADP) was used as a substrate, bacterial concentrations of strain GK1 could be measured down to $10^5$ bacteria/ml. In this experiment, NADP is converted to NAD in catalytic amounts. NADH is produced from NAD through the action of alcohol dehydrogenase and in the presence of ethanol. Diaphorase interconverts NADH to NAD, and at the same time a reducible product (INT) is reduced to a coloured product (21) (cf. Fig. 3).

EXAMPLE 3

Binding of anti-Gal-Gal-ALP beads to tlc-separated glycolipids

A mixture of globotetracylceramide, Gal$\alpha$1-4Gal$\beta$1-4Glc-$\beta$-O-(CH$_2$)$_{17}$CH$_3$, was applied to a HPTLC plate (Merck, Darmstadt, West Germany). The compounds were separated using chloroform-methanol-water (60:35:8, by volume) as the mobile phase. The plate was then covered by spraying with a thin film of methylmethacrylate (18). After drying, the exposed carbohydrate residues could be visualized by first incubating the plate with a water suspension of latex-anti-Gal-Gal-ALP beads (0.2 $\mu$m), and then washing the plate in water. The plate was covered with a Whatman filter paper soaked with p-nitrophenylphosphate. The reaction was complete after 15 minutes, and the narrow yellow bands could be detected by the naked eye.

EXAMPLE 4

Binding of (Gal-Gal)$_n$-ALP latex beads to antibodies immobilized on microtiter plates

Microtiter plates were coated by incubating them overnight at room temperature with 100 $\mu$l of anti-Gal-Gal antibody, diluted from 5 $\mu$g/ml to 0.17 $\mu$g/ml in 50 mmol NaHCO$_3$ buffer, pH 9.6. After washing, the wells were treated with a 50 mg/ml BSA solution, followed by washing. To each well was added a $10^8$ particle/ml suspension of (Gal-Gal)$_n$-ALP latex (0.81 $\mu$m, 7500 active ALP molecule/particle). After incubation for 0.5 hour and washing, the amount of microbeads bound was detected using p-nitrophenylphosphate as a substrate. The limit of detection was 0.17 $\mu$g/ml which corresponds to a concentration of approximately 0.2 pmol/l of this antibody solution (MW $10^6$).

A summary of the types of microbeads prepared and employed in the Examples and the results of agglutination tests with different *E. coli* strains is shown in the following Tables III and IV.

Table III

| Specificity and enzyme activity (ALP) of various beads | | | | |
|---|---|---|---|---|
| Modification of particles -coupling method | Size of particles | ALP units[*]/ $10^{10}$ part. | No. of active ALP molecules per part. | Area/ particle |
| Gal-gal-ALP, carbodiimide coupled to NH$_2$-latex | (0.21 $\mu$m) | 0.3 | 100 | 7x10$^{-6}$ |
| Gal-gal-ALP, carbodiimide coupled to BSA latex | (0.21 $\mu$m) | 0.3 | 95 | 7x10$^{-6}$ |
| ALP + gal-gal-BSA (10:1), carbodiimide coupled to NH$_2$-latex | (0.21 $\mu$m) | 1.0 | 380 | 27x10$^{-6}$ |
| ALP diazo-coupled to NH$_2$-latex, globoside-coated | (0.21 $\mu$m) | 0.2 | 80 | 6x10$^{-6}$ |
| Gal-gal-ALP, coated to latex | (0.81 $\mu$m) | 2.6 | 1000 | 5x10$^{-6}$ |
| ALP diazo-coupled to NH$_2$-latex, globoside-coated | (0.81 $\mu$m) | 52 | 20000 | 100x10$^{-6}$ |
| ALP covalently coupled to tosyl-activated Dynosphere, globoside-coated | (3 $\mu$m) | 17 | 6500 | 2x10$^{-6}$ |

Table IV

| Modification of particles - coupling method | Agglutination test of various beads (strain**) | | | | |
|---|---|---|---|---|---|
| | 1 (506MR) | 2 (GK1) | 3 (MAS11) | 4 (MAS4) | 5 (C600) |
| Gal-gal-ALP, carbodiimide coupled to $NH_2$-latex | + | ++ | + | 0 | + |
| Gal-gal-ALP, carbodiimide coupled to BSA latex | + | + | 0 | 0 | 0 |
| ALP + gal-gal-BSA (10:1), carbodiimide coupled to $NH_2$-latex | | +++ | | | + |
| ALP diazo-coupled to $NH_2$-latex, globoside-coated | | +++ | | | ++ |
| Gal-gal-ALP, coated to latex | | +++ | | | + |
| ALP diazo-coupled to $NH_2$-latex, globoside-coated | | +++ | | | ++ |
| ALP covalently coupled to tosyl-activated Dynosphere, globoside-coated | ++ | +++ | ++ | 0 | +(+) |

\* 1 µmol substrate/minute at 37°C, in diethanolamine buffer

\*\* Strains 1, 2 and 3 are identified as P-fimbriated *E. coli*, strains 4 and 5 are non-P-fimbriated.

0 = not binding
+ = weak binding
+ + = medium binding
+ + + = strong binding

22

EXAMPLE 5

Detection of *Staphylococcus saprophyticus* bound to polystyrene films coated with lactoseamine-BSA conjugate

It has previously been shown (12) in haemagglutination studies that *Staphylococcus saprophyticus* binds to lactose amine-containing glycoconjugates.

Transparent 0.2 mm thick Trysite films were coated with lactoseamine-BSA conjugate (100 $\mu$g/ml, see Example 1). After washing with Tris buffer, pH 7.4, the films were incubated for 0.5 hour with bacterial suspensions of *S. saprophyticus* ($10^{10}$-$10^8$ bacteria/ml). The films were carefully washed with water and Tris buffer, pH 7.4, and incubated with a microbead suspension ($10^8$ particles/ml, latex modified with lactoseamine-BSA/ALP; 5000 active ALP/particle). The films became opaque at bacterial concentrations of $10^{10}$ bacteria/ml. At lower concentrations, the specific binding was detected by a substrate incubation step as described in Example 1.

REFERENCES

1. S. Avrameas, *Curr. Top. Microbiol. Immunol. 104*, 1983, p. 93.

2. J.H. Caster, *J. Clin. Immunoassay 7*, 1984, p. 64.

3. C. Wagener et al., *J. Immunolog. Methods 68*, 1984, p. 269.

4. A.M. Shamsuddin and C.C. Harris, *Arch. Pathol. Lab. Med. 107*, 1983, p.514.

5. C. Bacquet and D.Y. Twumasi, *Anal. Biochem. 136*, 1984, p. 487.

6. R.H. Yolkan et al., *J. Immunolog. Methods 56*, 1983, p. 319.

7. C. Kendall et al., *J. Immunolog. Methods 56* 1983, p. 329.

8. A. Johansson, C.J. Stanley and C.H. Self, *Clinic Chimica Acta 148*, 1985, p. 119.

9. B. Poltsmann et al., *J. Immunolog. Methods 6*, 1984, p. 179.

10. J. Dahmén, T. Frejd, G. Grönberg, T. Lave, G. Magnusson and G. Noori, *Carbohydr. Res. 116*, 1983, p. 303.

11. J. Dahmén, T. Frejd, G. Grönberg, T. Lave, G. Magnusson and G. Noori, *Carbohydr. Res. 118*, 1983, p. 292.

12. J. Dahmén, T. Frejd, G. Magnusson, G. Noori and A.-S. Carlström, *Carbohydr. Res. 125*, 1984, p. 237.

13. H. Leffler and C. Svanborg-Edén, in *Bacterial Lectins*, ed. D. Mirelman, 1985.

14. H.J. Tenoso and D.B. Smith, *NASA TECH BRIEF*, Manned Spacecraft Centre (MSC 13906).

15. R. Möllby, G. Källenius, T.K. Korhonen, J. Winberg and S.B. Svensson, *Infection 11*, 1983, p. 68.

16. H. Leffler and C. Svanborg-Edén, *Infect. Immun. 34*, 1981, p. 920; G. Källenius, S.B. Svensson, R. Möllby, B. Cedergren, H. Hultberg and J. Winberg, *Lancet*, 1981, p. 604.

17. G.C. Hansson, K.-A. Karlsson, G. Larsson, N. Strömberg and J. Thurin, *Anal. Biochem. 146*, 1985, p. 158.

18. M. Dubois, K.A. Gilles, J.K. Hamilton, P.A. Rebers and F. Smith, *Anal. Chem. 28*, 1956, p. 350.

19. E. Engvall, *Methods in Enzymology 70*, p. 419.

20. C.H. Self, US Patent Application No. 307,600; A. Johannsson, C.J. Stanley and C.H. Self, *Clin. Chim. Acta 148*, 1985, p. 119.

21. A. Gunnarsson, P.-A. Märdh, A. Lundblad and S. Svensson, *Infect. and Immun. 45*, 1984.

**Claims**

1. A method of detecting the presence in a sample of a cell, virus, plasma protein, hormone or antibody, or an antibody thereto, characterised in that it comprises:

(a) contacting the sample with a first reagent bound to a solid support so that the reagent binds to the cell, virus, plasma protein, hormone or antibody, or antibody thereto;
(b) contacting the resulting sample containing the cell, virus, plasma protein, hormone or antibody, or antibody thereto bound to the reagent, which in turn is bound to the solid support, with a macromolecular water-insoluble carrier having a second reagent which is a carbohydrate or carbohydrate derivative and a label substance multivalently-bound thereto so that the second reagent and thus the macromolecular water-insoluble carrier and the label substance bind to the cell, virus, plasma protein, hormone or antibody, or antibody thereto;

(c) removing unbound second reagent and thus unbound carrier and label substance from the sample;

(d) treating the then-resulting sample with a compound which is a precursor of a detectable reaction product or which is capable of initiating a reaction cascade, the end product of which is detectable, so that a detectable reaction product is formed if the label substance is present; and

(e) detecting any detectable reaction product so formed and thereby detecting the presence of the cell, virus, plasma protein, hormone or antibody, or antibody thereto, in the sample.

2. A method as claimed in claim 1 wherein the first and second reagent are capable of binding a specific cell, virus, plasma protein, hormone or antibody.

3. A method as claimed in claim 1 or claim 2 wherein the first reagent and the second reagent are the same.

4. A method as claimed in any of claims 1 to 3 wherein the first reagent is selected from a carbohydrate or carbohydrate derivative such as a receptor for a cell, virus, plasma protein, hormone or antibody, e.g. a polysaccharide, glycolipid, neo-glycolipid, glycoprotein or neo-glycoprotein, a polypeptide, a protein or another cell or virus surface component, a plasma protein, hormone or antibody, a toxin, a cell membrane component, an antibody to a carbohydrate or carbohydrate derivative, a polypeptide, a protein or another cell or virus surface component or a plasma protein, hormone or antibody, a metal conjugate or a single-stranded oligonucleotide.

5. A method as claimed in any of claims 1 to 4 wherein the solid support to which the reagent used in (a) is bound comprises a polymer, e.g. a plastic, such as polystyrene, polyvinylchloride, polyethylene, polyurethane, latex, nylon, tetrafluoroethylene, polyethylene terephthalate, polyvinylacetate, polyvinylalcohol or a suitable copolymer thereof, a polysaccharide, a silicon polymer, an ion exchange resin, a polypeptide, silica or a silicate such as borosilicate, or cellulose, or wherein the solid support comprises a matrix to which the polymer is bound.

6. A method as claimed in any of claims 1 to 5 wherein the polymer comprises optionally modified functional groups.

7. A method as claimed in any of claims 1 to 6 wherein the solid support is in the form of a plate, film, macroparticle, dipstick, filter or paper.

8. A method as claimed in any of claims 1 to 7 wherein the sample comprises a pathogen, e.g. a bacterium, virus, fungus, mycoplasma or prion, or a bacterial toxin, a non-pathogen, a carbohydrate or carbohydrate derivative, a polypeptide, a protein or another cell or virus component, a toxin, a cell membrane component, an antibody to a carbohydrate or carbohydrate derivative, a polypeptide, a protein or another cell or virus component, or a single-stranded oligonucleotide which is complementary to that used as the reagent.

9. A method as claimed in any of claims 1 to 8 wherein the macromolecular water-insoluble carrier is a polymer containing multiple active sites, such as hydroxyl, amino, thio, amido, cyano, imino, imido, keto, epoxy, nitro, ester and acyl groups, or halide-containing groups, or wherein the macromolecular carrier is in the form of microbeads, e.g. microbeads comprising a polymer such as a plastic or a polysaccharide, a copolymer, a silicon polymer or a polymer containing functional groups which are optionally modified, a glass, a liposome, a cross-linked polymer or silica or a silicate, e.g. borosilicate, the microbeads optionally being provided with multiple active sites, e.g. hydroxyl, amino, thio, amido, cyano, imino, imido, keto, epoxy, nitro, ester or acyl groups, or halide containing groups.

10. A method as claimed in claim 9 wherein the microbeads have a size of from 0.05 to 20 $\mu$m.

11. A method as claimed in any of claims 1 to 10 wherein the substance which is multivalently bound to the macromolecular water-insoluble carrier is selected from enzymes, e.g. alkaline phosphatase, glucose oxidase, peroxidase, acid phosphatase, galactosidase, proteases, luciferase, dehydrogenase, esterase, urease and lysozyme, antibodies and blood clotting factors, e.g. fibrinogen (I), prothrombin (II), thrombin (IIa), tissue thromboplastin (III), proaccelerin (V), accelerator globulin (VI), proconvertin

24

(VII), antihemophilic globulin (VIII), plasma thromboplastin component (IX), autoprothrombin III (X), plasma thromboplastin antecedent (XI), Hagemann factor (XII) and fibrin-stabilizing factor (XIII).

12. A method as claimed in any of claims 1 to 11 wherein the compound which is a precursor of a detectable reaction product or is capable of initiating a reaction cascade the end product of which is detectable comprises a blood clotting factor, an antigen or an enzyme substrate which is catalyzed by the enzyme to form a coloured, fluorescent or luminescent product, or to produce a colour change, or to make the colour, fluorescence or luminescence of a product decrease or increase in intensity, e.g. phenolphthalein-orthophosphate, thymophthalein phosphate, orthonitrophenyl glycoside, naphthols, anilines, coumarins, tetrazolium dyes, dansyl and derivatives thereof, NADP or isothiocyanates.

13. A method as claimed in any of claims 1 to 7 or 9 to 12 wherein:

a) the solid support comprises macroparticles to which the first reagent is bound, which macroparticles are packed in a column, or the solid support comprises a porous filter to which the first reagent is bound, and the sample is passed through the column or filter;
(b) a suspension of water-insoluble microbeads of a size of from 1 to 20 $\mu$m to which the second reagent is coupled and to which an enzyme is multivalently-bound, is passed through the column; and
(c) a solution or suspension of enzyme substrate is passed through the column after which a colour, fluorescence or luminescence develops;

whereby the presence in the sample of any cell, virus, plasma protein, hormone or antibody which has become bound to the reagent in (a) and (b) is detected.

14. A method as claimed in any of claims 1 to 7 or 9 to 12 wherein:

(a) the solid support comprises a dipstick or film to which the reagent is bound, which dipstick or film is immersed in the sample;
b) the dipstick or film is contacted with the macromolecular carrier comprising microbeads of a size of from 0.05-10 $\mu$m to which the second reagent is coupled and to which an enzyme is multivalently-bound, by immersing it in a suspension of the microbeads, and
(c) the dipstick or film becomes opaque at high cell, virus, plasma protein, hormone or antibody concentrations, or the dipstick is immersed in a solution or suspension of enzyme substrate after which a colour, fluorescence or luminescence develops,

whereby the presence in the sample of a cell, virus, plasma protein, hormone or antibody which has become bound to the reagent in (a) and (b) is detected.

15. A method as claimed in any of claims 1 to 7 or 9 to 12 wherein:

(a) the solid support comprises a film or plate, optionally provided with wells, to which the reagent is bound, and the sample is applied on the film or plate and removed after a period of time sufficient to bind cell, virus, plasma protein, hormone or antibody in the sample;
(b) the macromolecular carrier comprises microbeads of a size of from 1 to 20 $\mu$m to which the second reagent is coupled and to which an enzyme is multivalently-bound, and a suspension of the microbeads is applied on the film or plate and removed after a period of time sufficient to bind cell, virus, plasma protein, hormone or antibody in the sample, and
(c) a solution or suspension of enzyme substrate is applied on the film or plate after which a colour, fluorescence or luminescence develops;

whereby the presence in the sample of a cell, virus, plasma protein, hormone or antibody which has become bound to the reagent in (a) and (b) is detected.

16. A method as claimed in any of claims 13 to 15, wherein the amount of cell, virus, plasma protein, hormone or antibody bound to the reagent in (a) is quantitized by means of measuring the amount of microbeads out of a predetermined quantity which are not bound to the cell, virus, plasma protein, hormone or antibody in the sample.

**17.** A macromolecular water-insoluble carrier suitable for use in a method as claimed in claim 1 characterised in that it is

(a) coupled to a reagent which is a carbohydrate or carbohydrate derivative, the reagent being capable of binding a cell, virus, plasma protein, hormone or antibody, or an antibody thereto, the carrier comprising a substance multivalently-bound to the carrier, which substance is capable of initiating a reaction the product of which is detectable; or
(b) multivalently-bound to a substance to which the reagent is coupled and which is capable of initiating a reaction the product of which is detectable.

**18.** A carrier as claimed in claim 17 wherein the reagent is capable of binding a specific cell or virus component.

**19.** A carrier as claimed in claim 17 or claim 18 wherein the reagent is selected from a carbohydrate or carbohydrate derivative, such as a receptor for a cell, virus, plasma protein, hormone or antibody, (e.g. a mono-, oligo- or poly-saccharide, glycolipid, neo-glycolipid, glycoprotein or neo-glycoprotein), a polypeptide, a protein or another cell or virus surface component, a plasma protein, hormone or antibody, a toxin, a cell membrane component or a single-stranded oligonucleotide.

**20.** A carrier as claimed in any of claims 17 to 19 wherein the macromolecular carrier is a polymer containing multiple active sites, such as hydroxyl, amino, thio, amido, cyano, imino, imido, keto, epoxy, nitro, ester and acyl groups, or halide containing groups, or in which the macromolecular carrier is in the form of microbeads, e.g. microbeads comprising a polymer, such as a plastic or a polysaccharide, a copolymer, a silicon polymer or a polymer containing functional groups which are optionally modified, a glass, a liposome, a cross-linked polymer or silica or a silicate e.g. borosilicate, the microbeads optionally being provided with multiple active sites, e.g. hydroxyl, amino, thio, amido, cyano, imino, imido, keto, epoxy, nitro, ester or acyl groups, or halide-containing groups.

**21.** A carrier as claimed in claim 20 wherein the microbeads have a size of from 0.05 to 20 $\mu$m.

**22.** A carrier as claimed in any of claims 17 to 21 wherein the substance to which the reagent is coupled or which is multivalently bound to the macromolecular carrier is selected from enzymes, e.g. alkaline phosphatase, glucose oxidase, peroxidase, acid phosphatase, galactosidase, proteases, luciferase, dehydrogenase, esterase, urease and lysozyme, antibodies and blood clotting factors, e.g. fibrinogen (I), prothrombin (II), thrombin (IIa), tissue thromboplastin (III), proaccelerin (V), accelerator globulin (VI), proconvertin (VII), antihemophilic globulin (VIII), plasma thromboplastin component (IX), autoprothrombin III (X), plasma thromboplastin antecedent (XI), Hagemann factor (XII) and fibrin-stabilizing factor (XIII).

**23.** A diagnostic composition suitable for use in a method as claimed in claim 1 characterised in that it comprises:

(a) a first reagent bound to a solid support, which reagent is capable of binding a cell, virus, plasma protein, hormone or antibody, or an antibody thereto, and
(b) a second reagent which is a carbohydrate or carbohydrate derivative, reagent being coupled to a macromolecular water-insoluble carrier either directly or via a spacer, to which carrier a label substance is multivalently-bound, said substance being capable of initiating a reaction the product of which is detectable, or coupled to a macromolecular water-insoluble carrier via a label substance, the substance being multivalently-bound to the macromolecular water-insoluble carrier and the substance being capable of initiating a reaction the product of which is detectable.

**24.** A composition as claimed in claim 23 wherein the reagent is capable of binding a specific cell, virus, plasma protein, hormone or antibody.

**25.** A composition as claimed in claim 23 or claim 24 wherein the first reagent and the second reagent are the same.

**26.** A composition as claimed in any of claims 23 to 25 wherein the second reagent is selected from a carbohydrate or carbohydrate derivative, such as a receptor for a cell, virus, plasma protein, hormone or antibody, (e.g. a mono-, oligo- or poly-saccharide, glycolipid, neo-glycolipid, glycoprotein or neo-glycoprotein), a polypeptide, a protein or another cell or virus surface component, a plasma protein, hormone or antibody, a toxin, a cell membrane component or a single-stranded oligonucleotide.

**27.** A composition as claimed in any of claims 23 to 26 wherein the solid support to which the reagent used in (a) is bound comprises a polymer, e.g. a plastic, such as polystyrene, polyvinylchloride, polyethylene, polyurethane, latex, nylon, tetrafluoroethylene, polyethylene terephthalate, polyvinylacetate, polyvinylalcohol or a suitable copolymer thereof, a polysaccharide, a silicon polymer, an ion exchange resin, a polypeptide, silica or a silicate, such as borosilicate, or cellulose, or in which the solid support comprises a matrix to which the polymer is bound.

**28.** A composition as claimed in claim 27 wherein the polymer comprises optionally modified functional groups.

**29.** A composition as claimed in any of claims 23 to 28 wherein the solid support is in the form of a plate, film, macroparticle, dipstick, filter or paper.

**30.** A composition as claimed in any of claims 23 to 29 wherein the macromomecular water-insoluble carrier is a polymer containing multiple active sites, such as hydroxyl, amino, thio, amido, cyano, imino, imido, keto, epoxy, nitro, ester and acyl groups, or halide containing groups, or in which the macromolecular water-insoluble carrier is in the form of microbeads, e.g. microbeads comprising a polymer, such as a plastic or a polysaccharide, a copolymer, a silicon polymer or a polymer containing functional groops which are optionally modified, a glass, a liposome, a cross-linked polymer or silica or a silicate e.g. borosilicate, the microbeads optionally being provided with multiple active sites, e.g. hydroxyl, amino, thio, amido, cyano, imino, imido, keto, epoxy, nitro, ester or acyl groups, or halide containing groups.

**31.** A composition as claimed in claim 30 wherein the microbeads have a size of from 0.05 to 20 $\mu$m.

**32.** A composition as claimed in any of claims 23 to 31 wherein the substance to which the second reagent may be coupled and which is multivalently-bound to the macromolecular carrier is selected from enzymes, e.g. alkaline phosphatase, glucose oxidase, peroxidase, acid phosphatase, galactosidase, proteases, luciferase, dehydrogenase, esterase, urease and lysozyme, antibodies and blood clotting factors, e.g. fibrinogen (I), prothrombin (II), thrombin (IIa), tissue thromboplastin (III), proaccelerin (V), acelerator globulin (VI), proconvertin (VII), antihemophilic globulin (VIII), plasma thromboplastin component (IX), autoprothrombin III (X), plasma thromboplastin antecedent (XI), Hagemann factor (XII) and fibrin-stabilizing factor (XIII).

**33.** A composition as claimed in any of claims 23 to 32 wherein the solid support comprises a multiplicity of dipsticks to which the reagent is bound, each dipstick or sub-group of dipsticks being provided with a different reagent, each reagent being able to bind a different cell, virus, plasma protein, hormone or antibody or an antibody thereto, or wherein the solid support comprises a microtiter plate provided with a multiplicity of wells to which the reagent is bound, each well or sub-group of wells containing a different reagent, each reagent being able to bind a different cell, virus, plasma protein, hormone or antibody or an antibody thereto.

**Revendications**

**1.** Procédé de détection de la présence dans un échantillon d'une cellule, d'un virus, d'une protéine plasmatique, d'une hormone ou d'un anticorps, ou d'un anticorps correspondant, caractérisé en ce qu'il comprend :
    (a) le contact de l'échantillon avec un premier réactif lié à un support solide, de façon à ce que la réactif se lie à la cellule, au virus, à la protéine plasmatique, à l'hormone ou à l'anticorps, ou à un anticorps correspondant ;
    (b) le contact de l'échantillon obtenu, contenant la cellule, le virus, la protéine plasmatique, l'hormone ou l'anticorps, ou un anticorps correspondant, lié au réactif qui lui-même est lié au support solide, avec un porteur macromoléculaire insoluble dans l'eau, ayant un second réactif qui est un glucide ou un dérivé glucidique, et une substance de marquage qui lui est liée par liaison plurivalente, si bien que le second réactif et donc le porteur macromoléculaire insoluble dans l'eau et la substance de marquage se lient à la cellule, au virus, à la protéine plasmatique, à l'hormone ou à l'anticorps, ou à un anticorps correspondant ;

(c) la séparation du second réactif non lié et donc du porteur non lié et de la substance de marquage de l'échantillon ;

(d) le traitement de l'échantillon ainsi obtenu avec un composé qui est un précurseur d'un produit réactionnel détectable ou qui est capable d'amorcer une cascade réactionnelle dont le produit final est détectable, si bien qu'un produit réactionnel détectable est formé lorsque la substance de marquage est présente ; et

(e) la détection de tout produit réactionnel détectable ainsi formé et ainsi la détection de la présence de la cellule, du virus, de la protéine plasmatique, de l'hormone ou de l'anticorps, ou d'un anticorps correspondant, dans l'échantillon.

**2.** Procédé selon la revendication 1, dans lequel le premier et le second réactifs sont capables de se lier à une cellule, un virus, une protéine plasmatique, une homone ou un anticorps spécifiques.

**3.** Procédé selon la revendication 1 ou la revendication 2, dans lequel le premier réactif et la second réactif sont les mêmes.

**4.** Procédé selon l'une quelconque des revendications 1 à 3, dans lequel le premier réactif est choisi parmi un glucide ou un dérivé glucidique, tel qu'un récepteur d'une cellule, d'un virus, d'une protéine plasmatique, d'une hormone ou d'un anticorps, par exemple un polysaccharide, un glycolipide, un néo-glycolipide, une glycoprotéine ou une néo-glycoprotéine, un polypeptide, une protéine ou un autre composant de la surface d'une cellule ou d'un virus, une protéine plasmatique, une hormone ou un anticorps, une toxine, un composant de membrane cellulaire, un anticorps contre un glucide ou un dérivé glucidique, un polypeptide, une protéine ou un autre composant du la surface d'une cellule ou d'un virus, ou une protéine plasmatique, une hormone ou un anticorps, un conjugué métallique ou un oligonucléotide monocaténaire.

**5.** Procédé selon l'une quelconque des revendications 1 à 4, dans lequel le support solide auquel le réactif utilisé en (a) est lié comprend un polymère, par exemple un plastique comme le polystyrène, le chlorure de polyvinyle, le polyéthylène, le polyuréthanne, un latex, le nylon, le tétrafluoroéthylène, le polyéthylène téréphtalate, le polyacétate de vinyle, l'alcool polyvinylique ou un copolymère approprié correspondant, un polysaccharide, un polymère silicié, une résine échangeuse d'ions, un polypeptide, la silice ou un silicate, tel qu'un borosilicate, ou la cellulose, et où le support solide comprend une matrice à laquelle le polymère est lié.

**6.** Procédé selon l'une quelconque des revendications 1 à 5, dans lequel le polymère comprend des groupes fonctionnels facultativement modifiés.

**7.** Procédé selon l'une quelconque des revendications 1 à 6, dans lequel le support solide est sous forme d'une plaque, d'une pellicule, d'une macroparticule, d'une bandelette, d'un filtre ou d'un papier.

**8.** Procédé selon l'une quelconque des revendications 1 à 7, dans lequel l'échantillon comprend un agent pathogène, par exemple une bactérie, un virus, un champignon, un mycoplasme ou un prion, ou une toxine bactérienne, un agent non pathogène, un glucide ou un dérivé glucidique, un polypeptide, une protéine ou un autre composant de cellule ou de virus, une toxine, un composant de membrane cellulaire, un anticorps contre un glucide ou un dérivé glucidique, un polypeptide, une protéine ou un autre composant de cellule ou de virus, ou un oligonucléotide monocaténaire qui est complémentaire de celui utilisé comme réactif.

**9.** Procédé selon l'une quelconque des revendications 1 à 8, dans lequel le porteur macromoléculaire insoluble dans l'eau est un polymère contenant des sites actifs multiples, tels que des groupes hydroxyles, amino, thio, amido, cyano, imino, imido, céto, époxy, nitro, esters et acyles, ou des groupes contenant un halogénure, ou dans lequel le porteur macromoléculaire est sous forme de microperles, par exemple de microperles comprenant un polymère tel qu'un plastique ou un polysaccharide, un copolymère, un polymère silicié ou un polymère contenant des groupes fonctionnels qui

28

sont facultativement modifiés, un verre, un liposome, un polymère réticulé ou une silice ou un silicate, par exemple un borosilicate, les microperles étant facultativement munies de sites actifs multiples, par exemple de groupes hydroxyles, amino, thio, amido, cyano, imino, imido, céto, époxy, nitro, esters ou acyles, ou des groupes contenant un halogénure.

**10.** Procédé selon la revendication 9, dans lequel les microperles ont une taille du 0,05 à 20 $\mu$m.

**11.** Procédé selon l'une quelconque des revendications 1 à 10, dans lequel la substance qui est liée par liaison plurivalente au porteur macromoléculaire insoluble dans l'eau est choisie parmi les enzymes, par exemple phospatase alcaline, glucose oxydase, peroxydase, phosphatase acide, galactosidase, protéases, luciférase, déshydrogénase, estérase, uréase et lysozyme, des anticorps et des facteurs de la coagulation du sang, par exemple le fibrinogène (I), la prothrombine (II), la thrombine (IIa), la thromboplastine tissulaire (III), la proaccélérine (V), l'accélérine (VI), la proconvertine (VII), la globuline antihémophilique (VIII), le composant de thromboplastine plasmatique (IX), l'autoprothrombine III (X), le précurseur de la thromboplastine plasmatique (XI), le facteur Hageman (XII) et le facteur stabilisant de la fibrine (XIII).

**12.** Procédé selon l'une quelconque des revendications 1 à 11, dans lequel le composé, qui est un précurseur d'un produit réactionnel détectable ou qui est capable d'amorcer une cascade réactionnelle dont le produit final est détectable, comprend un facteur de la coagulation du sang, un antigène ou un substrat enzymatique qui est catalysé par l'enzyme pour former un produit coloré, fluorescent ou luminescent ou pour produire un changement de couleur ou pour accroître ou réduire l'intensité de la couleur, de la fluorescence ou de la luminescence d'un produit, par exemple l'orthophosphate de phénolphtaléine, le phosphate de thymolphtaléine, un orthonitrophénylglycoside, des naphtols, des anilines, des coumarines, des colorants de type tétrazolium, le dansyle et ses dérivés, le NADP ou les isothiocyanates.

**13.** Procédé' selon l'une quelconque des revendications 1 à 7 ou 9 à 12, dans lequel :
(a) le support solide comprend des macroparticules auxquelles le premier réactif est lié, lesquelles macroparticules garnissent une colonne, ou le support solide comprend un filtre poreux auquel le premier réactif est lié et l'échantillon traverse la colonne ou le filtre ;
(b) une suspension de microperles insolubles dans l'eau ayant une taille de 1 à 20 $\mu$m, auxquelles le second réactif est couplé et auxquelles une enzyme est liée par liaison plurivalente, traverse la colonne ; et
(c) une solution ou suspension de substrat enzymatique traverse la colonne, après quoi une couleur, une fluorescence ou une luminescence s'établit,
si bien que la présence dans l'échantillon d'une cellule, d'un virus, d'un plasma, d'une protéine, d'une hormone ou d'un anticorps qui se sont liés au réactif en (a) et (b) est détectée.

**14.** Procédé selon l'une quelconque des revendications 1 à 7 ou 9 à 12, dans lequel :
(a) le support solide comprend une bandelette ou une pellicule auxquelles le réactif est lié, laquelle bandelette ou laquelle pellicule est plongée dans l'échantillon ;
(b) la bandelette ou la pellicule est mise en contact avec le porteur macromoléculaire comprenant des microperles, ayant une taille de 0,05 à 10 $\mu$m, auxquelles le second réactif est couplé et auxquelles une enzyme est liée par liaison plurivalente, par immersion dans une suspension des microperles et
(c) la bandelette ou la pellicule devient opaque pour des concentrations élevées en cellule, en virus, en protéine plasmatique, en hormone ou en anticorps, ou la bandelette est plongée dans une solution ou suspension de substrat enzymatique, après quoi une couleur, une fluorescence ou une luminescence s'établit,
si bien que la présence dans l'échantillon d'une cellule, d'un virus, d'une protéine plasmatique, d'une hormone ou d'un anticorps qui se sont liés au réactif en (a) et (b) est détectée.

**15.** Procédé selon l'une quelconque des revendications 1 à 7 ou 9 à 12, dans lequel :
(a) le support solide comprend une pellicule ou une plaque, éventuellement munie de cupules, à laquelle le réactif est lié et l'échantillon est appliqué à la pellicule ou à la plaque et séparé après une période suffisante pour lier la cellule, le virus, la protéine plasmatique, l'hormone ou l'anticorps de l'échantillon,

(b) le porteur macromoléculaire comprend des microperles ayant une taille de 1 à 20 $\mu$m auxquelles le second réactif est couplé et auxquelles une enzyme est liée par liaison plurivalente, et une suspension des microperles est appliquée à la pellicule ou à la plaque et séparée après une période suffisante pour lier la cellule, le virus, la protéine plasmatique, l'hormone ou l'anticorps de l'échantillon et

(c) une solution ou suspension de substrat enzymatique est appliquée à la pellicule ou à la plaque, après quoi une couleur, une fluorescence ou une luminescence s'établit ;

si bien que la présence dans l'échantillon d'une cellule, d'un virus, d'une protéine plasmatique, d'une hormone ou d'un anticorps qui se sont liés au réactif en (a) et (b) est détectée.

16. Procédé selon l'une quelconque des revendications 13 à 15, dans lequel la quantité de cellule, de virus, de protéine plasmatique, d'hormone ou d'anticorps liée au réactif en (a) est déterminée quantitativement par mesure de la fraction des microperles d'une quantité prédéterminée qui ne sont pas liées à la cellule, au virus, à la protéine plasmatique, à l'hormone ou à l'anticorps de l'échantillon.

17. Porteur macromoléculaire insoluble dans l'eau approprié à l'emploi dans un procédé selon la revendication 1, caractérisé en ce que

(a) il est couplé à un réactif qui est un glucide ou un dérivé glucidique, le réactif étant capable de se lier à une cellule, à un virus, à une protéine plasmatique, à une hormone ou à un anticorps, ou à un anticorps correspondant, le porteur comprenant une substance liée par liaison plurivalente au porteur, laquelle substance est capable d'amorcer une réaction dont le produit est détectable ; ou

(b) il est lié par liaison plurivalente à une substance à laquelle le réactif est couplé et qui est capable d'amorcer une réaction dont le produit est détectable.

18. Porteur selon la revendication 17, dans lequel le réactif est capable de se lier à un composant cellulaire ou viral spécifique.

19. Porteur selon la revendication 17 ou la revendication 18, dans lequel le réactif est choisi parmi un glucide ou un dérivé glucidique, tel qu'un récepteur d'une cellule, d'un virus, d'une protéine plasmatique, d'une hormone ou d'un anticorps (par exemple un mono-, oligo- ou polysaccharide, un glycolipide, un néo-glycolipide, une glycoprotéine ou une néo-glycoprotéine), un polypeptide, une protéine ou un autre composant de la surface d'une cellule ou d'un virus, une protéine plasmatique, une hormone ou un anticorps, une toxine, un composant de membrane cellulaire ou un oligonucléotide monocaténaire.

20. Porteur selon l'une quelconque des revendications 17 à 19, dans lequel le porteur macromoléculaire est un polymère contenant des sites actifs multiples, tels que des groupes hydroxyles, amino, thio, amido, cyano, imino, imido, céto, époxy, nitro, esters et acyles, ou des groupes contenant un halogénure, ou dans lequel le porteur macromoléculaire est sous forme de microperles, par exemple de microperles comprenant un polymère tel qu'un plastique ou un polysaccharide, un copolymère, un polymère silicié ou un polymère contenant des groupes fonctionnels qui sont facultativement modifiés, un verre, un liposome, un polymère réticulé ou une silice ou un silicate, par exemple un borosilicate, les microperles étant facultativement munies de sites actifs multiples, par exemple de groupes hydroxyles, amino, thio, amido, cyano, imino, imido, céto, époxy, nitro, esters ou acyles, ou des groupes contenant un halogénure.

21. Porteur selon la revendication 20, dans lequel les microperles ont une taille de 0,05 à 20 $\mu$m.

22. Porteur selon l'une quelconque des revendications 17 à 21, dans lequel la substance qui est liée par liaison plurivalente au porteur macromoléculaire insoluble dans l'eau est choisie parmi les enzymes, par exemple phosphatase alcaline, glucose oxydase, peroxydase, phosphatase acide, galactosidase, protéases, luciférase, déshydrogénase, estérase, uréase et lysozyme, des anticorps et des facteurs de la coagulation du sang, par exemple la fibrinogène (I), la prothrombine (II), la thrombine (IIa), la thromboplastine tissulaire (III), la proaccélérine (V), l'accélérine (VI), la proconvertine (VII), la globuline antihémophilique (VIII), le composant de thromboplastine plasmatique (IX), l'autoprothrombine III (X), le précurseur de la thromboplastine plasmatique (XI), le facteur Hageman (XII) et le facteur stabilisant de la fibrine (XIII).

23. Composition de diagnostic appropriée à l'emploi dans un procédé selon la revendication 1, caractérisée en ce qu'elle comprend :

(a) un premier réactif lié à un support solide, lequel réactif est capable de se lier à une cellule, à un virus, à une protéine plasmatique, à une hormone ou à un anticorps, ou à un anticorps correspondant et

(b) un second réactif qui est un glucide ou un dérivé glucidique, le réactif étant couplé à un porteur macromoléculaire insoluble dans l'eau, soit directement, soit via un espaceur, auquel porteur une substance de marquage est liée par une liaison plurivalente, ladite substance étant capable de déclencher une réaction dont le produit est détectable, ou couplé à un porteur macromoléculaire insoluble dans l'eau via une substance de marquage, la substance étant liée par liaison plurivalente au porteur macromoléculaire insoluble dans l'eau et la substance étant capable d'amorcer une réaction dont le produit est détectable.

24. Composition selon la revendication 23, dans laquelle le réactif est capable de se lier à une cellule, à un virus, à une protéine plasmatique, à une hormone ou à un anticorps spécifiques.

25. Composition selon la revendication 23 ou la revendication 24, dans laquelle le premier réactif et le second réactif sont les mêmes.

26. Composition selon l'une quelconque des revendications 23 à 25, dans laquelle le second réactif est choisi parmi un glucide ou un dérivé glucidique, tel qu'un récepteur d'une cellule, d'un virus, d'une protéine plasmatique, d'une hormone ou d'un anticorps (par exemple un mono-, oligo- ou polysaccharide, un glycolipide, un néo-glycolipide, une glycoprotéine ou une néo-glycoprotéine), un polypeptide, une protéine ou un autre composant de la surface d'une cellule ou d'un virus, une protéine plasmatique, une hormone ou un anticorps, une toxine, un composant de membrane cellulaire ou un oligonucléotide monocaténaire.

27. Composition selon l'une quelconque des revendications 23 à 26, dans laquelle le support solide auquel le réactif utilisé en (a) est lié comprend un polymère, par exemple un plastique comme le polystyrène, la chlorure de polyvinyle, le polyéthylène, le polyuréthanne, un latex, le nylon, la tétrafluoroéthylène, le polyéthylène téréphtalate, le polyacétate de vinyle, l'alcool polyvinylique ou un copolymère approprié correspondant, un polysaccharide, un polymère silicié, une résine échangeuse d'ions, un polypeptide, la silice ou un silicate, tel qu'un borosilicate, ou la cellulose, ou dans laquelle le support solide comprend une matrice à laquelle le polymère est lié.

28. Composition selon la revendication 27, dans laquelle le polymère comprend des groupes fonctionnels facultativement modifiés.

29. Composition selon l'une quelconque des revendications 23 à 28, dans laquelle le support solide est sous forme d'une plaque, d'une pellicule, d'une macroparticule, d'une bandelette, d'un filtre ou d'un papier.

30. Composition selon l'une quelconque des revendications 23 à 29, dans laquelle le porteur macromoléculaire insoluble dans l'eau est un polymère contenant des sites actifs multiples, tels que des groupes hydroxyles, amino, thio, amido, cyano, imino, imido, céto, époxy, nitro, esters et acyles, ou des groupes contenant un halogénure, ou dans laquelle le porteur macromoléculaire insoluble dans l'eau est sous forme de microperles, par exemple de microperles comprenant un polymère tel qu'un plastique ou un polysaccharide, un copolymère, un polymère silicié ou un polymère contenant des groupes fonctionnels qui sont facultativement modifiés, un verre, un liposome, un polymère réticulé ou une silice ou un silicate, par exemple un borosilicate, les microperles étant facultativement munies de sites actifs multiples, par exemple de groupes hydroxyles, amino, thio, amido, cyano, imino, imido, céto, époxy, nitro, esters ou acyles, ou des groupes contenant un halogénure.

31. Composition selon la revendication 30, dans laquelle les microperles ont une taille de 0,05 à 20 $\mu$m.

32. Composition selon l'une quelconque des revendications 23 à 31, dans laquelle la substance à laquelle le second réactif peut être couplé et qui est liée par liaison plurivalente au porteur macromoléculaire est choisie parmi les enzymes, par exemple phosphatase alcaline, glucose oxydase, peroxydase, phosphatase acide, galactosidase, protéases, luciférase, déshydrogénase, estérase, uréase et lysozy-

EP 0 241 140 B1

me, des anticorps et des facteurs de la coagulation du sang, par exemple la fibrinogène (I), la prothrombine (II), la thrombine (IIa), la thromboplastine tissulaire (III), la proaccélérine (V), l'accélérine (VI), la proconvertine (VII), la globuline antihémophilique (VIII), le composant de thromboplastine plasmatique (IX), l'autoprothrombine III (X), le précurseur de la thromboplastine plasmatique (XI), le facteur Hageman (XII) et le facteur stabilisant de la fibrine (XIII).

33. Composition selon l'une quelconque des revendications 23 à 32, dans laquelle le support solide comprend plusieurs bandelettes auxquelles la réactif est lié, chaque bandelette ou sous-groupe de bandelettes étant muni d'un réactif différent, chaque réactif étant capable de se lier à une cellule, à un virus, à une protéine plasmatique, à une hormone ou à un anticorps, ou à un anticorps correspondant différents, ou dans laquelle le support solide comprend une plaque de microtitrage munie de plusieurs cupules auxquelles le réactif est lié, chaque cupule ou sous-groupe de cupules contenant un réactif différent, chaque réactif étant capable de se lier à une cellule, à un virus, à une protéine plasmatique, à une hormone ou à un anticorps, ou à un anticorps correspondant différents.

**Patentansprüche**

1. Ein Verfahren zum Nachweis der Anwesenheit einer Zelle, eines Virus, eines Plasmaproteins, eines Hormons oder Antikörpers, oder eines dagegen gerichteten Antikörpers in einer Analysenprobe, **dadurch gekennzeichnet,** daß es umfaßt:

(a) Inkontaktbringen der Probe mit einem ersten Reagenz, das an einen festen Träger gebunden ist, so daß das Reagenz an die Zelle, das Virus, das Plasmaprotein, das Hormon oder den Antikörper, oder den dagegen gerichteten Antikörper bindet,

(b) Inkontaktbringen der erhaltenen Analysenprobe, enthaltend die Zelle, das Virus, das Plasmaprotein, das Hormon oder den Antikörper, oder den dagegen gerichteten Antikörper, gebunden an das Reagenz, das wiederum an den festen Träger gebunden ist, mit einem makromolekularen, wasserunlöslichen Trägermaterial, das ein zweites Reagenz, welches ein Kohlenhydrat oder Kohlenhydratderivat ist und eine Markierungssubstanz multivalent daran gebunden enthält, so daß das zweite Reagenz und somit das makromolekulare, wasserunlösliche Trägermaterial und die Markierungssubstanz an die Zelle, das Virus, das Plasmaprotein, das Hormon oder den Antikörper, oder den dagegen gerichteten Antikörper bindet;

(c) Entfernen von nicht gebundenem zweiten Reagenz und somit von ungebundenem Trägermaterial und Markierungssubstanz aus der Analysenprobe;

(d) Behandeln der dann erhaltenen Analysenprobe mit einer Verbindung, die ein Vorläufer eines nachweisbaren Reaktionsproduktes ist, oder die eine Reaktionskaskade einleiten kann, wobei deren Endprodukt nachweisbar ist, so daß ein nachweisbares Reaktionsprodukt gebildet wird, wenn die Markierungssubstanz vorhanden ist; und

(e) Nachweisen eines jeden nachweisbaren so gebildeten Reaktionsprodukts und dadurch Nachweisen der Anwesenheit der Zelle, des Virus, des Plasmaproteins, des Hormons oder Antikörpers, oder des dagegen gerichteten Antikörpers in der Analysenprobe.

2. Ein Verfahren wie in Anspruch 1 beansprucht, worin das erste und zweite Reagenz eine spezifische Zelle, Virus, Plasmaprotein, Hormon oder Antikörper binden kann.

3. Ein Verfahren wie in Anspruch 1 oder Anspruch beansprucht, worin das erste Reagenz und das zweite Reagenz das gleiche sind.

4. Ein Verfahren wie in einem der Ansprüche 1 bis 3 beansprucht, worin das erste Reagenz ausgewählt wird aus einem Kohlenhydrat oder Kohlenhydratderivat, wie einem Rezeptor für eine Zelle, ein Virus, ein Plasmaprotein, ein Hormon oder einen Antikörper, z.B. ein Polysaccharid, ein Glycolipid, ein Neo-Glycolipid, ein Glycoprotein oder Neo-Glycoprotein, einem Polypeptid, einem Protein oder einem anderen Zell- oder Virusoberflächenbestandteil, einem Plasmaprotein, einem Hormon oder einem Antikörper, einem Toxin, einem Zellmembranbestandteil, einem Antikörper gegen ein Kohlenhydrat oder ein Kohlenhydratderivat, ein Polypeptid, ein Protein oder einen anderen Zell- oder Virusoberflächenbestandteil oder ein Plasmaprotein, ein Hormon oder Antikörper, einem Metallkonjugat oder einem einzelsträngigen Oligonucleotid.

32

**5.** Ein Verfahren wie in einem der Ansprüche 1 bis 4 beansprucht, worin der feste Träger, an den das in (a) verwendete Reagenz gebunden ist, umfaßt ein Polymer, z.B. einen Kunststoff, wie Polystyrol, Polyvinylchlorid, Polyethylen, Polyurethan, Latex, Nylon, Tetrafluorethylen, Polyethylenterephthalat, Polyvinylacetat, Polyvinylalkohol oder ein geeignetes Copolymer davon, ein Polysaccharid, ein Silikonpolymer, ein Ionenaustauscherharz, ein Polypeptid, Silica oder ein Silicat, wie Borsilicat oder Zellulose, oder worin der feste Träger eine Matrix umfaßt, an die das Polymer gebunden ist.

**6.** Ein Verfahren wie in einem der Ansprüche 1 bis 5 beansprucht, worin das Polymer gegebenenfalls modifizierte funktionelle Gruppen umfaßt.

**7.** Ein Verfahren wie in einem der Ansprüche 1 bis 6 beansprucht, worin der feste Träger in der Form einer Platte, eines Films, eines Makropartikels, eines Meßstabs, eines Filters oder eines Papiers vorliegt.

**8.** Ein Verfahren wie in einem der Ansprüche 1 bis 7 beansprucht, worin die Analysenprobe umfaßt ein Pathogen, z.B. ein Bakterium, ein Virus, einen Pilz, Mycoplasmen oder ein Prion, oder ein bakterielles Toxin, ein Nichtpathogen, ein Kohlenhydrat oder Kohlenhydratderivat, ein Polypeptid, ein Protein oder einen anderen Zell- oder Virusbestandteil, ein Toxin, einen Zellmembranbestandteil, einen Antikörper gegen ein Kohlenhydrat oder Kohlenhydratderivat, ein Polypeptid, ein Protein oder einen anderen Zell- oder Virusbestandteil, oder ein einzelsträngiges Oligonucleotid, das komplementär zu dem als Reagenz verwendeten ist.

**9.** Ein Verfahren wie in einem der Ansprüche 1 bis 8 beansprucht, worin das makromolekulare, wasserunlösliche Trägermaterial ein Polymer ist, enthaltend mehrere aktive Stellen, wie Hydroxyl-, Amino-, Thio-, Amido-, Cyano-, Imino-, Imido-, Keto-, Epoxy-, Nitro-, Ester- und Acylgruppen, oder halogenhaltige Gruppen, oder worin das makromolekulare Trägermaterial in Form von Mikrokügelchen, z.B. Mikrokügelchen umfassend ein Polymer, wie einen Kunststoff oder ein Kohlenhydrat, ein Copolymer, ein Siliconpolymer oder ein Polymer, enthaltend funktionelle Gruppen, die gegebenenfalls modifiziert sind, ein Glas, ein Liposom, ein vernetztes Polymer oder Silica oder ein Silicat, z.B. Borsilicat, vorliegt, wobei die Mikrokügelchen gegebenenfalls mit mehreren aktiven Stellen, wie Hydroxyl-, Amino-, Thio-, Amido-, Cyano-, Imino-, Imido-, Keto-, Epoxy-, Nitro-, Ester- oder Acylgruppen, oder halogenhaltigen Gruppen ausgestattet sind.

**10.** Ein Verfahren wie in Anspruch 9 beansprucht, worin die Mikrokügelchen eine Größe von 0,05 bis 20 $\mu$m besitzen.

**11.** Ein Verfahren wie in einem der Ansprüche 1 bis 10 beansprucht, worin die Substanz, die multivalent an das makromolekulare, wasserunlösliche Trägermaterial gebunden ist, ausgewählt wird aus Enzymen, z.B. alkalische Phosphatase, Glucoseoxidase, Peroxidase, saure Phosphatase, Galactosidase, Proteasen, Luciferase, Dehydrogenase, Esterase, Urease und Lysozym, Antikörpern und Blutgerinnungsfaktoren, z.B. Fibrinogen (I), Prothrombin (II), Thrombin (IIa), Gewebsthromboplastin (III), Proaccelerin (V), Acceleratorglobulin (VI), Proconvertin (VII), antihemophiles Globulin (VIII), Plasmathromboplastinbestandteil (IX), Autoprothrombin III (X), Plasmathromboplastinvorläufer (XI), Hagemann-Faktor (XII) und fibrinstabilisierender Faktor (XIII).

**12.** Ein Verfahren wie in einem der Ansprüche 1 bis 11 beansprucht, worin die Verbindung, die ein Vorläufer eines nachweisbaren Reaktionsprodukts ist, oder die eine Reaktionskaskade einleiten kann, von der das Endprodukt nachweisbar ist, umfaßt einen Blutgerinnungsfaktor, ein Antigen oder ein Enzymsubstrat, das von dem Enzym umgesetzt wird, um ein farbiges, fluoreszierendes oder lumineszierendes Produkt zu bilden, oder um eine Farbänderung zu erzeugen, oder die Intensität der Farbe, Fluoreszenz oder Lumineszenz eines Produkts zu verringern oder zu steigern, z.B. Phenolphthalein-Orthophosphat, Thymolphthaleinphosphat, o-Nitrophenylglycosid, Naphthole, Aniline, Coumarine, Tetrazoliumfarbstoffe, Dansyl oder Derivate davon, NADP oder Isothiocyanate.

**13.** Ein Verfahren wie in einem der Ansprüche 1 bis 7 oder 9 bis 12 beansprucht, worin:

(a) der feste Träger Makropartikel umfaßt, an die das erste Reagenz gebunden ist, wobei die Makropartikel in eine Säule gepackt sind, oder der feste Träger umfaßt einer porösen Filter, an den

33

das erste Reagenz gebunden ist, und die Analysenprobe wird durch die Säule oder den Filter geleitet;

(b) eine Suspension aus wasserunlöslichen Mikrokügelchen mit einer Größe von 1 bis 20 μm, an die das zweite Reagenz gekoppelt ist und an die ein Enzym multivalent gebunden ist, durch die Säule geleitet wird; und

(c) eine Lösung oder Suspension von Enzymsubstrat durch die Säule geleitet wird, wonach sich eins Farbe, Fluoreszenz oder Lumineszenz entwickelt;

wodurch die Anwesenheit einer beliebigen Zelle, eines Virus, eines Plasmaproteins, eines Hormons oder eines Antikörpers, die an das Reagenz in (a) und (b) gebunden haben, nachgewiesen wird.

**14.** Ein Verfahren wie in einem der Ansprüche 1 bis 7 oder 9 bis 12 beansprucht, worin:

(a) der feste Träger einen Meßstab oder Film umfaßt, an den das Reagenz gebunden ist, wobei der Meßstab oder Film in die Analysenprobe eingetaucht wird;

(b) der Maßstab oder Film mit dem makromolekularen Trägermaterial, umfassend Mikrokügelchen einer Größe von 0,05-10 μm, an die das zweite Reagenz gekoppelt ist und an die ein Enzym multivalent gebunden ist, in Kontakt gebracht wird durch Eintauchen in eine Suspension der Mikrokügelchen; und

(c) der Meßstab oder Film undurchsichtig wird bei hohen Zell-, Virus-, Plasmaprotein-, Hormon- oder Antikörperkonzentrationen, oder der Meßstab wird in eine Lösung oder Suspension von Enzymsubstrat eingetaucht, wonach sich eine Farbe, Fluoreszenz oder Lumineszenz entwickelt, wodurch die Anwesenheit einer Zelle, eines Virus, eines Plasmaproteins, eines Hormons oder Antikörpers, die an das Reagenz in (a) und (b) gebunden worden sind, nachgewiesen wird.

**15.** Ein Verfahren wie in einem der Ansprüche 1 bis 7 oder 9 bis 12 beansprucht, worin:

(a) der feste Träger einen Film oder eine Platte, gegebenenfalls mit Vertiefungen ausgestattet, umfaßt, an den bzw. die das Reagenz gebunden ist, und die Analysenprobe wird auf den Film oder die Platte gegeben und wird nach einer Zeitspanne, die ausreicht, die Zelle, das Virus, das Plasmaprotein, das Hormon oder den Antikörper in der Probe zu binden, entfernt;

(b) das makromolekulare Trägermaterial Mikrokügelchen mit einer Größe von 1 bis 20 μm umfaßt, an die das zweite Reagenz gekoppelt ist und an die ein Enzym multivalent gebunden ist, und eine Suspension der Mikrokügelchen wird auf den Film oder die Platte gegeben und nach einer Zeitspanne, die ausreicht, um die Zelle, das Virus, das Plasmaprotein, das Hormon oder den Antikörper in der Probe zu binden, entfernt, und

(c) eine Lösung oder eine Suspension von Enzymsubstrat auf den Film oder die Platte gegeben wird, wonach sich eine Farbe, Fluoreszenz oder Lumineszenz entwickelt,

wodurch die Anwesenheit einer Zelle, eines Virus, eines Plasamproteins, eines Hormons oder Antikörpers, das an das Reagenz in (a) und (b) gebunden worden ist, nachgewiesen wird.

**16.** Ein Verfahren wie in einem der Ansprüche 13 bis 15 beansprucht, worin die Menge an das Reagenz in (a) gebundenen Zellen, des Virus, des Plasmaproteins, des Hormons oder Antikörpers quantifiziert wird durch Messen der Menge von Mikrokügelchen aus einer vorab bestimmten Menge, die nicht an die Zelle, das Virus, das Plasmaprotein, das Hormon oder den Antikörper in der Analysenprobe gebunden ist.

**17.** Ein makromolekulares, wasserunlösliches Trägermaterial, das zur Verwendung in einem Verfahren, wie in Anspruch 1 beansprucht, geeignet ist, **dadurch gekennzeichnet,** daß es

(a) an ein Reagenz gekoppelt ist, das ein Kohlenhydrat oder Kohlenhydratderivat ist, wobei das Reagenz eins Zelle, ein Virus, ein Plasmaprotein, ein Hormon oder einen Antikörper oder einen dagegen gerichteten Antikörper binden kann, wobei das Trägermaterial eine multivalent an das Trägermaterial gebundene Substanz umfaßt, wobei die Substanz eine Reaktion einleiten kann, deren Produkt nachweisbar ist oder

(b) multivalent an eine Substanz gebunden ist, an die das Reagenz gekoppelt ist, und die eine Reaktion einleiten kann, deren Produkt nachweisbar ist.

**18.** Ein Trägermaterial wie in Anspruch 17 beansprucht, worin das Reagenz einen spezifischen Zell- oder Virusbestandteil binden kann.

**19.** Ein Trägermaterial wie in Anspruch 17 oder Anspruch 18 beansprucht, worin das Reagenz ausgewählt wird aus einem Kohlenhydrat oder Kohlenhydratderivat, wie einem Rezeptor für eine Zelle, ein Virus, ein Plasmaprotein, ein Hormon oder einen Antikörper (z.B. ein Mono-, oligo- oder Polysaccharid, Glycolipid, Neo-Glycolipid, Glycoprotein oder Neo-Glycoprotein), einem Polypeptid, einem Protein oder einem anderen Zell- oder Virusoberflächenbestandteil, einen Plasmaprotein, einem Hormon oder Antikörper, einem Toxin, einem Zellmembranbestandteil oder einem einzelsträngigen Oligonucleotid.

**20.** Ein Trägermaterial wie in einem der Ansprüche 17 bis 19 beansprucht, worin das makromolekulare Trägermaterial ein Polymer ist, enthaltend mehrere aktive Stellen, wie Hydroxyl-, Amino-, Thio-, Amido-, Cyano-, Imino, Imido-, Keto-, Epoxy-, Nitro-, Ester- und Acylgruppen, oder halogenhaltige Gruppen, oder in dem das makromolekulare Trägermaterial in der Form von Mikrokügelchen, z.B. Mikrokügelchen umfassend ein Polymer, wie einen Kunststoff oder ein Polysaccharid, ein Copolymer, ein Siliconpolymer oder ein Polymer, enthaltend funktionelle Gruppen, die gegebenenfalls modifiziert sind, ein Glas, ein Liposom, ein vernetztes Polymer oder Silica oder ein Silicat, z. B. Borsilicat, vorliegt, wobei die Mikrokügelchen gegebenenfalls mit mehreren aktiven Stellen, z.B. Hydroxyl-, Amino-, Thio-, Amido-, Cyano-, Imino-, Imido-, Keto-, Epoxy-, Nitro-, Ester- oder Acylgruppen, oder halogenhaltigen Gruppen ausgestattet sind.

**21.** Ein Trägermaterial wie in Anspruch 20 beansprucht, worin die Mikrokügelchen eine Größe Von 0,5 - 20 μm besitzen.

**22.** Ein Träger wie in einem der Ansprüche 17 bis 21 beansprucht, worin die Substanz, an die das Reagenz gekoppelt ist, oder die multivalent an das makromolekulare Trägermaterial gebunden ist, ausgewählt wird aus Enzymen, z.B. alkalische Phosphatase, Glucoseoxidase, Peroxidase, saure Phosphatase, Galactosidase, Proteasen, Luciferase, Dehydrogenase, Esterase, Urease und Lysozym, Antikörpern und Blutgerinnungsfaktoren, z.B. Fibrinogen (I), Prothrombin (II), Thrombin (IIa), Gewebsthromboplastin (III), Proaccelerin (V), Acceleratorglobulin (VI), Proconvertin (VII), antihemophiles Globulin (VIII), Plasmathromboplastinbestandteil (IX), Autoprothrombin III (X), Plasmathromboplastinvorläufer (XI), Hagemann-Faktor (XII) und fibrinstabilisierender Faktor (XIII).

**23.** Eine Zusammensetzung zur Diagnostik, die zur Verwendung in einem wie in Anspruch 1 beanspruchten Verfahren geeignet ist, **dadurch gekennzeichnet,** daß sie umfaßt:

(a) ein erstes an einen festen Träger gebundenes Reagenz, wobei das Reagenz eine Zelle, ein Virus, ein Plasmaprotein, ein Hormon oder einen Antikörper, oder einen dagegen gerichteten Antikörper binden kann, und
(b) ein zweites Reagenz, das ein Kohlenhydrat oder Kohlenhydratderivat ist, wobei das Reagenz an ein makromolekulares, wasserunlösllches Trägermaterial entweder direkt oder über einen Abstandshalter gekoppelt ist, wobei an das Trägermaterial eine Markierungssubstanz multivalent gebunden ist, wobei die Substanz eine Reaktion einleiten kann, deren Produkt nachweisbar ist, oder wobei das Reagenz gekoppelt ist an ein makromolekulares, wasserunlösliches Trägermaterial über eine Markersubstanz, wobei die Substanz multivalent an das makromolekulare, wasserunlösliche Trägermaterial gebunden ist, und die Substanz eine Reaktion einleiten kann, deren Produkt nachweisbar ist.

**24.** Eine Zusammensetzung wie in Anspruch 23 beansprucht, worin das Reagenz eine spezifische Zelle, ein Virus, ein Plasmaprotein, ein Hormon oder einen Antikörper binden kann.

**25.** Eine Zusammensetzung wie in Anspruch 23 oder 24 beansprucht, worin das erste Reagenz und das zweite Reagenz das gleiche sind.

**26.** Eine Zusammensetzung wie in einem der Ansprüche 23 bis 25 beansprucht, worin das zweite Reagenz ausgewählt wird aus einem Kohlenhydrat oder Kohlenhydratderivat, wie einem Rezeptor für eine Zelle, ein Virus, ein Plasmaprotein, ein Hormon oder einen Antikörper (z.B. ein Mono-, Oligo- oder Polysaccharid, Glycolipid, Neo-Glycolipid, Glycoprotein oder Neo-Glycoprotein), einem Polypeptid, einem Protein oder einem anderen Zell- oder Virusoberflächenbestandteil, einem Plasmaprotein, einem Hormon oder einem Antikörper, einem Toxin, einem Zellmembranbestandteil oder einem einzelsträngigen Oligonucleotid.

**27.** Eine Zusammensetzung wie in einem der Ansprüche 23 bis 26 beansprucht, worin der feste Träger, an den das in (a) verwendete Reagenz gebunden ist, umfaßt ein Polymer, z.B. einen Kunststoff, wie Polystyrol, Polyvinylchlorid, Polyethylen, Polyurethan, Latex, Nylon, Tetrafluorethylen, Polyethylenterephthalat, Polyvinylacetat, Polyvinylalkohol oder ein geeignetes Copolymer davon, ein Polysaccharid, ein Siliconpolymer, ein Ionenaustauscherharz, ein Polypeptid, Silica oder ein Silicat, wie Borsilicat, oder Zellulose, oder in der der Teste Träger eine Matrix umfaßt, an die das Polymer gebunden ist.

**28.** Eine Zusammensetzung wie in Anspruch 27 beansprucht, worin das Polymer gegebenenfalls modifizierte funktionelle Gruppen umfaßt.

**29.** Eine Zusammensetzung wie in einem der Ansprüche 23 bis 28 beansprucht, worin der feste Träger in der Form einer Platte, eines Films, eines Makropartikels, eines Meßstabs, eines Filters oder Papiers vorliegt.

**30.** Eine Zusammensetzung wie in einem der Ansprüche 23 bis 29 beansprucht, worin das makromolekulare, wasserunlösliche Trägermaterial ein Polymer ist, enthaltend mehrere aktive Stellen, wie Hydroxyl-, Amino-, Thio-, Amido-, Cyano-, Imino-, Imido-, Keto-, Epoxy-, Nitro-, Ester- und Acylgruppen, oder halogenhaltige Gruppen, oder in der das makromolekulare, wasserunlösliche Trägermaterial in der Form von Mikrokügelchen vorliegt, z.B. Mikrokügelchen umfassend ein Polymer, wie einen Kunststoff oder ein Polysaccharid, ein Copolymer, ein Silikonpolymer oder ein Polymer enthaltend funktionelle Gruppen, die gegebenenfalls modifiziert sind, ein Glas, ein Liposom, ein vernetztes Polymer oder Silica oder ein Silicat, z.B. Borsilicat, wobei die Mikrokügelchen gegebenenfalls mit mehreren aktiven Stellen, z.B. Hydroxyl-, Amino-, Thio-, Amido-, Cyano-, Imino-, Imido-, Keto-, Epoxy-, Nitro-, Ester- oder Acylgruppen, oder halogenhaltigen Gruppen ausgestattet sind.

**31.** Eine Zusammensetzung wie in Anspruch 30 beansprucht, worin die Mikrokügelchen eine Größe von 0,05 bis 20 $\mu$m besitzen.

**32.** Eine Zusammensetzung wie in einem der Ansprüche 23 bis 31 beansprucht, worin die Substanz, an die das zweite Reagenz gekoppelt sein kann und die multivalent an das makromolekulare Trägermaterial gebunden ist, ausgewählt wird aus Enzymen, z.B. alkalische Phosphatase, Glucoseoxidase, Peroxidase, saure Phosphatase, Galactosidase, Proteasen, Luciferase, Dehydrogenase, Esterase, Urease und Lysozym, Antikörpern und Blutgerinnungsfaktoren, z.B. Fibrinogen (I), Prothrombin (II), Thrombin (IIa), Gewebsthromboplastin (III), Proaccelerin (V), Acceleratorglobulin (VI), Proconvertin (VII), antihemophiles Globulin (VIII), Plasmathromboplastinbestandteil (IV), Autoprothrombin III (X), Plasmathromboplastinvorläufer (XI), Hagemann-Faktor (XII) und fibrinstabilisierender Faktor (XIII).

**33.** Eine Zusammensetzung wie in einem der Ansprüche 23 bis 32 beansprucht, worin der feste Träger eine Vielzahl von Meßstäben enthält, an die das Reagenz gebunden ist, wobei jeder Meßstab oder Untergruppe von Meßstäben mit einem unterschiedlichen Reagenz ausgestattet ist, wobei jedes Reagenz eine verschiedene Zelle, Virus, Plasmaprotein, Hormon oder Antikörper oder einen dagegen gerichteten Antikörper binden kann, oder worin der feste Träger eine Mikrotiterplatte umfaßt, die mit einer Vielzahl von Vertiefungen ausgestattet ist, an die das Reagenz gebunden ist, wobei jede Vertiefung oder Untergruppe von Vertiefungen ein verschiedenes Reagenz enthält, wobei jedes Reagenz eine verschiedene Zelle, Virus, Plasmaprotein, Hormon oder Antikörper oder einen dagegen gerichteten Antikörper binden kann.

Fig. 1.

Fig. 2.

Fig. 3.